# EUROPEAN PATENT SPECIFICATION

(11) **EP 3 931 349 B1**
(45) Date of publication and mention of the grant of the patent: **11.12.2024**
(21) Application number: 20763347.0
(22) Date of filing: 28.02.2020
(51) Int. Cl.: C12Q 1/6886, A61K 31/195, A61K 31/337, A61K 45/06, A61P 43/00

(54) **APOE GENOTYPING IN CANCER PROGNOSTICS AND TREATMENT**
APOE-GENOTYPING IN DER KREBSPROGNOSE UND -THERAPIE
GÉNOTYPAGE APOE DANS LE PRONOSTIC ET LE TRAITEMENT DU CANCER

(30) Priority: 28.02.2019 US 201962811575 P
(43) Date of publication of application: 05.01.2022
(73) Proprietor: Inspirna, Inc., Long Island City, NY 11101 (US)
(72) Inventor: TAVAZOIE, Sohail F., New York, NY 10065 (US); OSTENDORF, Benjamin N., New York, NY 10021 (US); TAVAZOIE, Masoud, New York, NY 10065 (US); GONSALVES, Foster, Brooklyn, NY 11238 (US)
(74) Representative: Barker Brettell LLP
(86) International application number: PCT/US2020/020349
(87) International publication number: WO 2020/176846

(56) References cited:
- WO-A1-2015/065505
- WO-A1-2017/161188
- WO-A1-95/16791
- WO-A2-2014/028461
- WO-A2-2017/181163
- CN-A- 108 738 323
- CN-A- 109 251 974
- US-A- 5 945 289
- US-A1- 2006 160 079
- US-A1- 2012 231 457
- US-A1- 2015 065 515
- US-A1- 2015 150 941
- US-A1- 2017 066 791
- US-A1- 2018 072 810
- BENN MARIANNE ET AL: "Low-Density Lipoprotein Cholesterol and the Risk of Cancer: A Mendelian Randomization Study", JOURNAL OF THE NATIONAL CANCER INSTITUTE, vol. 103, no. 6, 1 February 2011 (2011-02-01), GB, pages 508 - 519, XP055972059, ISSN: 0027-8874, DOI: 10.1093/jnci/djr008
- SPEIDELL ANDREW P ET AL: "Development of a HumanAPOEKnock-in Mouse Model for Study of Cognitive Function After Cancer Chemotherapy", NEUROTOXICITY RESEARCH, HARWOOD ACADEMIC PUBLISHERS, LAUSANNE, CH, vol. 35, no. 2, 4 October 2018 (2018-10-04), pages 291 - 303, XP036674838, ISSN: 1029-8428, [retrieved on 20181004], DOI: 10.1007/S12640-018-9954-7
- CHANG N W ET AL: "Apolipoprotein E4 allele influences the response of plasma triglyceride levels to tamoxifen in breast cancer patients", CLINICA CHIMICA ACTA, ELSEVIER BV, AMSTERDAM, NL, vol. 401, no. 1-2, 1 March 2009 (2009-03-01), pages 144 - 147, XP025898593, ISSN: 0009-8981, [retrieved on 20081211], DOI: 10.1016/J.CCA.2008.12.005
- SIMONS KARIN H ET AL: "T cell co-stimulation and co-inhibition in cardiovascular disease: a double-edged sword", NATURE REVIEWS CARDIOLOGY, NATURE PUBLISHING GROUP, GB, vol. 16, no. 6, 15 February 2019 (2019-02-15), pages 325 - 343, XP036784140, ISSN: 1759-5002, [retrieved on 20190215], DOI: 10.1038/S41569-019-0164-7
- FYMAT ALAIN: "Harnessing the Immune System to Treat Cancers and Neurodegenerative Diseases", 4 April 2018 (2018-04-04), XP055853404, Retrieved from the Internet <URL:https://asclepiusopen.com/clinical-research-in-neurology/volume-1-issue-1/8.pdf> [retrieved on 20211021]
- YANG ET AL.: "Causal relevance of circulating high-density lipoprotein cholesterol with cancer: a Mendelian randomization meta-analysis", SCI REP, vol. 5, no. 9495, 30 March 2015 (2015-03-30), pages 9495, XP055764108
- TAVAZOIE ET AL.: "LXR/ApoE Activation Restricts Innate Immune Suppression in Cancer", CELL, vol. 172, no. 4, 11 January 2018 (2018-01-11), pages 825 - 840, XP085347108
- VERGHESE ET AL.: "ApoE influences amyloid-.beta. (A.beta.) clearance despite minimal apoE/A.beta. association in physiological conditions", PROCEEDINGS OF THE NATIONAL ACADEMY OF SCIENCES, vol. 110, no. 19, 7 May 2013 (2013-05-07), pages E1807 - E1816, XP055485641

## Description

### CROSS-REFERENCE TO RELATED APPLICATIONS

This application claims priority of U.S. Provisional Application No. 62/811,575, filed on February 28, 2019.

### FIELD OF THE INVENTION

This invention relates generally to methods and reagents for cancer prognosis and treatment and more specifically to methods and reagents for cancer prognosis and treatment based on APOE genotyping.

### BACKGROUND OF THE INVENTION

Cancer remains one of the leading causes of death in the United States. Clinically, a broad variety of medical approaches, including surgery, radiation therapy, and chemotherapeutic drug therapy are currently being used in the treatment of human cancer. However, it is recognized that such approaches continue to be limited by a fundamental inability to accurately predict the likelihood of metastasis and tumor recurrence or the most efficacious treatment regime for minimizing the occurrence of these negative outcomes.

The discovery and clinical validation of markers for cancer of all types which can predict prognosis, likelihood of invasive or metastatic spread, is one of the major challenges facing oncology today. In breast cancer, for example, 70% of the approximately 186,000 annual cases present as lymph node-negative; however, 30% of these cases will recur after local therapy (mastectomy or "lumpectomy") (Boring et al. Clin. J. Cancer 42: 19-38 (1992)). It remains uncertain how to best identify patients whose risk of disease recurrence exceeds their risk of significant therapeutic toxicity (Osbourne, J. Clin. Oncol. 10: 679-682 (1992)), in order to decide the appropriate therapy for these patients.

Thus, there remains a strong need for developing methods and reagents for cancer treatment and cancer prognosis to determine adjuvant therapy, tumor surveillance, and the likelihood of disease progression. Benn M., et al. (2011-02-01) discloses a method of treating cancer with statin in a individuals genotyped for APOE R112C (rs429358) and R158C (rs7412) polymorphisms. Speidell A., et al. (2018-10-04) relates to female APOE3 and APOE4 homozygous mice that were either left untreated or treated with the most commonly used breast cancer therapeutic agent, doxorubicin. WO 2015/065505 A1 (UNIV DUKE [US]) is directed to a method for preventing or treating a subject suffering from or at risk of suffering from an estrogen dependent cancer. Intratumoral concentration of 27-hydroxy cholesterol decreased upon treatment with GW273297X in E0771 tumor bearing APOE3 mice. Chang N.W., et al. (2009-03-01) relates to thirty-three women with breast cancer that were recruited to examine the APOE polymorphism and fasting plasma lipid profiles before and after tamoxifen treatment for 6 months.US 2015/150941 A1 discloses a methods for identifying a subject responsive to treatment with a chemotherapeutic agent, wherein the subject is afflicted with chronic lymphocytic leukemia, including detecting the presence or absence of at least one APOE4 allele in a biological sample of the subject, wherein the presence of at least one APOE4 allele identifies the subject as a subject whose chronic lymphocytic leukemia is responsive to treatment with the chemotherapeutic agent.

### SUMMARY OF INVENTION

This disclosure addresses the need mentioned above in a number of aspects. In one aspect, this disclosure provides a method of treating cancer in a subject having at least one ε4 allele from polymorphisms at rs7412 or rs429358 or ε3 alleles from polymorphisms at rs7412 and rs429358 in the *ApoE* gene on chromosome 19. The method comprises administering to the subject an effective amount of an LXRβ agonist, or a pharmaceutically acceptable salt thereof.

In another aspect, this disclosure provides a method of treating cancer in a subject. The method comprises: (a) providing a subject identified as having at least one ε4 allele from polymorphisms at rs7412 or rs429358 or ε3 alleles from polymorphisms at rs7412 and rs429358 in the *ApoE* gene on chromosome 19; and (b) administering to the subject an effective amount of an LXRβ agonist, or a pharmaceutically acceptable salt thereof.

In some embodiments, the method comprises: (a) providing a subject identified as having at least one ε4 allele from polymorphisms at rs7412 or rs429358 or ε3 alleles from polymorphisms at rs7412 and rs429358 in the *ApoE* gene on chromosome 19 and identified as likely to benefit from treatment with an LXRβ agonist, or a pharmaceutically acceptable salt thereof, based on the presence of the at least one ε4 allele from polymorphisms at rs7412 or rs429358 or ε3 alleles from polymorphisms at rs7412 and rs429358; and (b) administering to the identified subject an effective amount of the LXRβ agonist, or a pharmaceutically acceptable salt thereof.

In some embodiments, the method comprises (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with an LXRβ agonist, or a pharmaceutically acceptable salt thereof; and (d) administering an effective amount of the LXRβ agonist, or a pharmaceutically acceptable salt thereof, to the identified subject, whereby the cancer is treated.

In another aspect, this disclosure also provides a method of identifying an individual having a cancer who may benefit from treatment with an LXRβ agonist, or a pharmaceutically acceptable salt thereof. The method comprises: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; and (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with an LXRβ agonist, or a pharmaceutically acceptable salt thereof, thereby identifying the individual having a cancer who may benefit from treatment with an LXRβ agonist, or a pharmaceutically acceptable salt thereof.

In some embodiments, the LXRβ agonist can be 2-[3-[(3R)-3-[[2-chloro-3-(trifluoromethyl)phenyl]methyl-(2,2-diphenylethyl)amino]butoxy]phenyl]acetic acid having the structure: 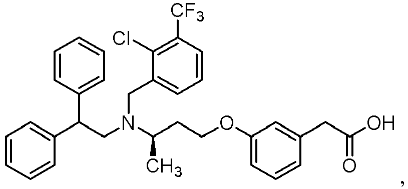 or a pharmaceutically acceptable salt thereof.

In another aspect, this disclosure further provides a method of treating cancer in a subject having at least one ε4 allele from polymorphisms at rs7412 or rs429358 or ε3 alleles from polymorphisms at rs7412 and rs429358 in the *ApoE* gene on chromosome 19. The method comprises administering an effective amount of a PD-1 inhibitor (e.g., nivolumab, pembrolizumab, pidilizumab, BMS 936559, and atezolizumab) or PD-L1 inhibitor (e.g., atezolizumab and durvalumab), or a pharmaceutically acceptable salt thereof, to the subject.

In another aspect, this disclosure provides a method of treating cancer in a subject in need thereof. The method comprises: (a) providing a subject identified as having at least one ε4 allele from polymorphisms at rs7412 or rs429358 or ε3 alleles from polymorphisms at rs7412 and rs429358 in the *ApoE* gene on chromosome 19; and (b) administering to the subject an effective amount of a PD-1 inhibitor (*e.g.,* nivolumab, pembrolizumab, pidilizumab, BMS 936559, and atezolizumab) or PD-L1 inhibitor (*e.g.,* atezolizumab and durvalumab), or a pharmaceutically acceptable salt thereof.

In some embodiments, the method comprises: (a) providing a subject identified as having at least one ε4 allele from polymorphisms at rs7412 or rs429358 or ε3 alleles from polymorphisms at rs7412 and rs429358 in the *ApoE* gene on chromosome 19 and identified as likely to benefit from treatment with a PD-1 inhibitor or PD-L1 inhibitor, or a pharmaceutically acceptable salt thereof, based on the presence of the at least one ε4 allele from polymorphisms at rs7412 or rs429358 or ε3 alleles from polymorphisms at rs7412 and rs429358; and (b) administering an effective amount of the PD-1 inhibitor or PD-L1 inhibitor to the identified subject.

In some embodiments, the method comprises: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with a PD-1 inhibitor or PD-L1 inhibitor, or a pharmaceutically acceptable salt thereof; and (d) administering an effective amount of the PD-1 inhibitor or PD-L1 inhibitor, or a pharmaceutically acceptable salt thereof, to the identified subject, whereby the cancer is treated.

In some embodiments, this disclosure provides a method of identifying an individual having a cancer who may benefit from treatment with a PD-1 inhibitor or PD-L1 inhibitor. The method comprises: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; and (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with a PD-1 inhibitor or PD-L1 inhibitor, or a pharmaceutically acceptable salt thereof, thereby identifying the individual having a cancer who may benefit from treatment with a PD-1 inhibitor or PD-L1 inhibitor, or a pharmaceutically acceptable salt thereof.

In some embodiments, the subject has been previously administered a CTLA-4 inhibitor, or a pharmaceutically acceptable salt thereof.

In some embodiments, the presence of at least one ε4 allele from polymorphisms at rs7412 or rs429358 or ε3 alleles from polymorphisms at rs7412 and rs429358 in the *ApoE* gene on chromosome 19 is determined by Polymerase Chain Reaction (PCR).

In some embodiments, the presence of at least one ε4 allele from polymorphisms at rs7412 or rs429358 or ε3 alleles from polymorphisms at rs7412 and rs429358 in the *ApoE* gene on chromosome 19 is determined by the expression level of APOE3 protein or APOE4 protein in the subject.

In some embodiments, the cancer is breast cancer, colon cancer, renal cell cancer, lung cancer, hepatocellular carcinoma, gastric cancer, ovarian cancer, pancreatic cancer, esophageal cancer, prostate cancer, sarcoma, bladder cancer, head and neck cancer, glioblastoma, diffuse large B-cell lymphoma, leukemia, or melanoma. In some embodiments, the cancer is melanoma, small cell lung cancer, or non-small cell lung cancer. In some embodiments, the cancer is metastatic and/or locally advanced. In some embodiments, the cancer is unresectable.

In some embodiments, the effective amount comprises an amount effective to suppress metastatic colonization of the cancer (e.g., metastatic colonization to the liver and/or brain).

In some embodiments, the cancer is resistant to platinum-containing chemotherapy, a PD-1 inhibitor, a PD-L1 inhibitor, a CTLA-4 inhibitor, an antimitotic agent, a topoisomerase inhibitor, an antimetabolite, an angiogenesis inhibitor, a kinase inhibitor, and/or an alkylating agent. In some embodiments, the cancer progressed on or after treatment with platinum-containing chemotherapy, a PD-1 inhibitor, a PD-L1 inhibitor, an angiogenesis inhibitor, a kinase inhibitor, and/or an alkylating agent. In some embodiments, the cancer has been determined to be, or is predicted to be, resistant to a PD-1 inhibitor, a PD-L1 inhibitor, a CTLA-4 inhibitor, a topoisomerase inhibitor, an antimetabolite, an angiogenesis inhibitor, a kinase inhibitor, and/or an alkylating agent.

In some embodiments, the method further comprises administering an additional anti-cancer therapy to the subject. In some embodiments, the additional anti-cancer therapy comprises surgery, radiation, chemotherapy, and/or immunotherapy. In some embodiments, the additional anti-cancer therapy comprises chemotherapy and/or immunotherapy. In some embodiments, the chemotherapy comprises docetaxel, carboplatin or cisplatin, pemetrexed, and/or pembrolizumab.

In some embodiments, the method further comprises administering to the subject an effective amount of folic acid, vitamin B12, corticosteroids, a statin, an anti-emetic agent, an anti-diarrheal agent, an appetite stimulant, a general stimulant, a bisphosphonate, a gonadotrophin-releasing hormone agonist, growth factors, and/or an LHRH agonist.

In some embodiments, the subject has ε3 alleles from polymorphisms at rs7412 and rs429358 in the *ApoE* gene. In some embodiments, the subject has at least one ε4 allele from polymorphisms at rs7412 or rs429358 in the *ApoE* gene. In some embodiments, the subject has ε4 alleles from polymorphisms at rs7412 and rs429358, an ε3 allele and an ε4 allele from polymorphisms at rs7412 and rs429358, or an ε2 and an ε4 allele from polymorphisms at rs7412 and rs429358 in the *ApoE* gene. In some embodiments, the subject has ε4 alleles from polymorphisms at rs7412 and rs429358 in the *ApoE* gene.

In yet another aspect, this disclosure further provides a method of treating cancer in a subject having at least one ε2 allele from polymorphisms at rs7412 or rs429358 in the *ApoE* gene on chromosome 19. The method comprises administering an aggressive treatment to the subject.

In another aspect, this disclosure provides a method of treating cancer in a subject in need thereof. The method comprises: (a) providing a subject identified as having at least one ε2 allele from polymorphisms at rs7412 or rs429358 in the *ApoE* gene on chromosome 19; and (b) administering to the subject an aggressive treatment.

In some embodiments, the method comprises: (a) providing a subject identified as having at least one ε2 allele from polymorphisms at rs7412 or rs429358 in the *ApoE* gene on chromosome 19 and identified as likely to benefit from an aggressive treatment based on the presence of the at least one ε2 allele from polymorphisms at rs7412 or rs429358; and (b) administering an aggressive treatment to the identified subject.

In some embodiments, the method comprises: (a) obtaining a sample containing nucleic acid from the subject; (b) performing a genotyping assay on the sample and detecting the presence of at least one ε2 allele from polymorphisms at rs7412 or rs429358 in the *ApoE* gene on chromosome 19; (c) identifying the subject having the at least one ε2 allele from polymorphisms at rs7412 or rs429358 in the *ApoE* gene on chromosome 19 as having an increased likelihood of benefiting from an aggressive treatment; and (d) administering an aggressive treatment to the identified subject, whereby the cancer is treated.

In another aspect, this disclosure provides a method of identifying an individual having a cancer who may benefit from an aggressive treatment. The method comprises: (a) obtaining a sample containing nucleic acid from the subject; (b) performing a genotyping assay on the sample and detecting the presence of at least one ε2 allele from polymorphisms at rs7412 or rs429358 in the *ApoE* gene on chromosome 19; and (c) identifying the subject having the at least one ε2 allele from polymorphisms at rs7412 or rs42935 8 in the *ApoE* gene on chromosome 19 as having an increased likelihood of benefiting from an aggressive treatment, thereby identifying the individual having a cancer who may benefit from an aggressive treatment.

In some embodiments, the presence of at least one ε2 allele from polymorphisms at rs7412 or rs429358 in the *ApoE* gene on chromosome 19 is determined by PCR. In some embodiments, the presence of at least one ε2 allele from polymorphisms at rs7412 or rs429358 in the *ApoE* gene on chromosome 19 is determined by the expression level of APOE2 protein in the subject.

In some embodiments, the subject has ε2 alleles from polymorphisms at rs7412 and rs429358 or an ε2 allele and an ε3 allele from polymorphisms at rs7412 and rs429358 in the *ApoE* gene on chromosome 19.

In some embodiments, the aggressive treatment comprises administering 2-[3-[(3R)-3-[[2-chloro-3-(trifluoromethyl)phenyl]methyl-(2,2-diphenylethyl)amino]butoxy]phenyl]acetic acid, or a pharmaceutically acceptable salt thereof and an immunotherapy in amounts that together are effective.

In some embodiments, the aggressive treatment further comprises performing surgery on the subject. In some embodiments, the 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof is administered prior to the surgery. In some embodiments, the 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof is administered after the surgery.

In some embodiments, the immunotherapy is a PD-1 inhibitor, a PD-L1 inhibitor, or a CTLA-4 inhibitor.

In another aspect, this disclosure additionally provides a kit comprising a probe having a first nucleic acid sequence complementary to a second nucleic acid sequence selected from the group consisting of APOE2, APOE3, and APOE4, wherein the probe hybridizes to the second nucleic acid sequence under a stringent condition. In some embodiments, the first nucleic acid sequence is 15-200 bases in length.

In some embodiments, the kit comprises labeled probes having sequences that are complementary to APOE2, APOE3, and APOE4, respectively. In some embodiments, the second nucleic acid comprises a sequence selected from the group consisting of SEQ ID NOs: 1-3.

In some embodiments, the kit further comprises a pair of primers configured to specifically bind sequences flanking a genomic region to allow PCR amplification of at least one of APOE2, APOE3, and APOE4 harboring the genomic region. In some embodiments, the pair of primers comprises a primer having a sequence selected from the group consisting of SED ID NOs: 4-16.

In some embodiments, the probe is linked with a detection label. In some embodiments, the detection label is selected from the group consisting of a fluorophore, a dye, a radiolabel, an enzyme, and a biotin.

In yet another aspect, this disclosure also provides an array comprising a support having a plurality of unique locations, wherein the array consists essentially of at least a nucleic acid having a sequence complementary to a nucleic acid sequence selected from the group consisting of APOE2, APOE3, and APOE4. In some embodiments, the nucleic acid sequence is selected from the group consisting of SEQ ID NOs: 1-3.

The foregoing summary is not intended to define every aspect of the disclosure, and additional aspects are described in other sections, such as the following detailed description. The entire document is intended to be related as a unified disclosure, and it should be understood that all combinations of features described herein are contemplated, even if the combination of features are not found together in the same sentence, or paragraph, or section of this document. Other features and advantages of the invention will become apparent from the following detailed description. It should be understood, however, that the detailed description and the specific examples, while indicating specific embodiments of the disclosure, are given by way of illustration only, because various changes and modifications within the scope of the disclosure will become apparent to those skilled in the art from this detailed description.

### BRIEF DESCRIPTION OF THE DRAWINGS

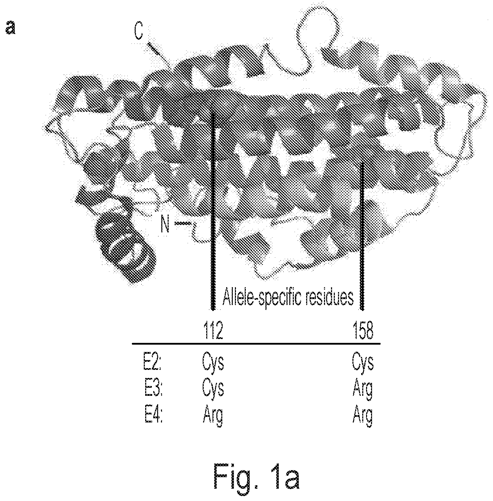

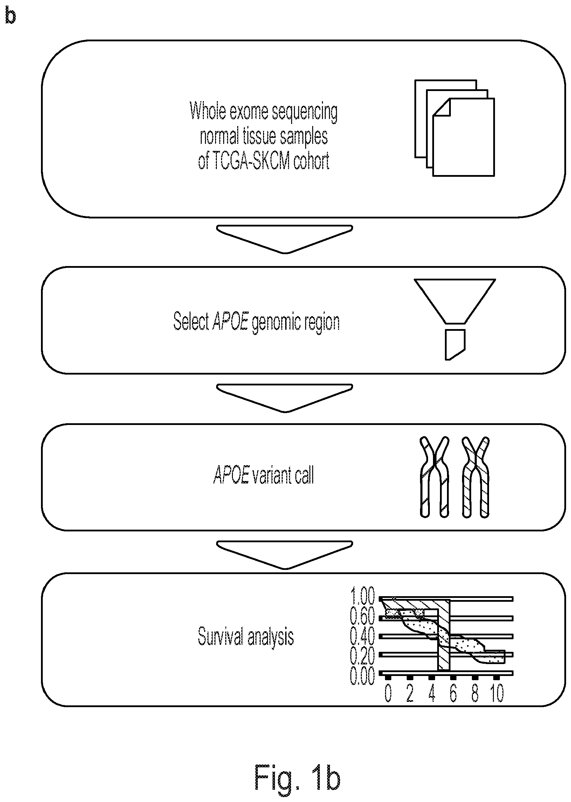

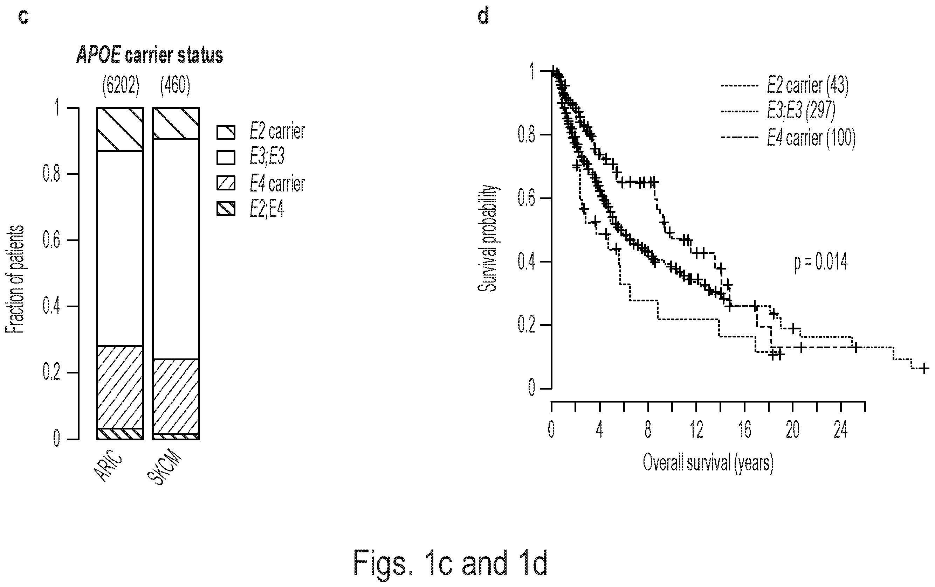

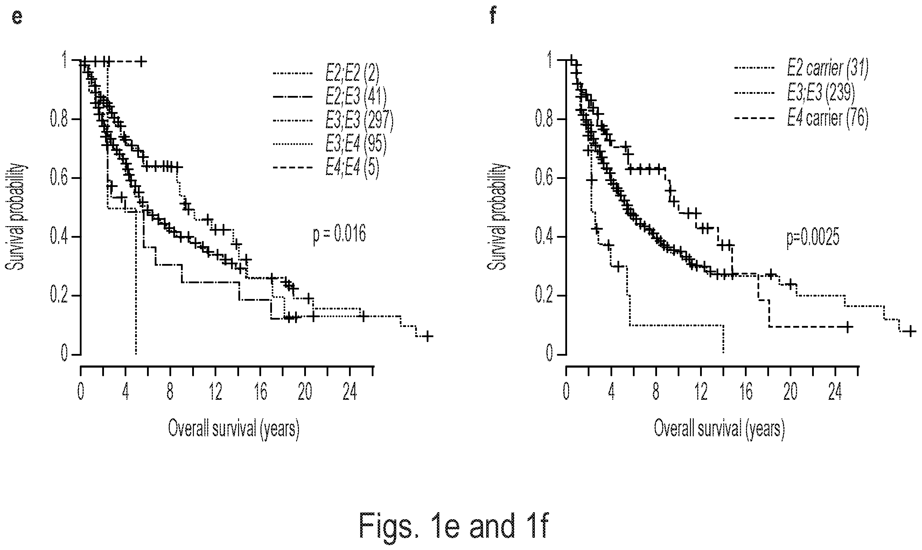

FIGs. 1a, 1b, 1c, 1d, 1e, and 1f are a set of diagrams showing that Apolipoprotein E (APOE) germline variants were used to predict survival in human melanoma. FIG. 1a shows two amino acids that define three prevalent APOE variants, APOE2, APOE3, and APOE4. Structural representation of human APOE3 (PDB ID: 2L7B; Chen et al. PNAS, 2011). The presence of either cysteine or arginine in positions 112 and 158 differentiates the alleles APOE2 (abbreviated as E2), APOE3 (abbreviated as E2), and APOE4 (abbreviated as E2). FIG. 1b shows the computational pipeline for analyzing the impact of APOE genotype on outcome in melanoma patients in the TCGA-SKCM cohort. FIG. 1c shows the proportion of E2 and E4 carriers in the Atherosclerosis Risk in Communities Study (ARIC) and the TCGA-SKCM cohorts. As indicated, non-E2/E4 carriers were modestly overrepresented in the TCGA-SKCM cohort versus a healthy population (P = 0.0017, χ² test). FIG. 1d shows APOE carrier status predicted survival in melanoma patients. Survival of the TCGA-SKCM cohort stratified by APOE carrier status (P= 0.014, log-rank test for trend). FIG. 1e shows that APOE variants exert a gene dosage effect on melanoma survival. Survival of the TCGA- SKCM cohort stratified by bi-allelic APOE genotype (P= 0.016, log-rank test for trend). FIG. 1f shows that APOE carrier status was associated with survival in patients with advanced localized disease. Survival of stage II-III patients in the TCGA-SKCM cohort stratified by APOE carrier status (P= 0.0025, log-rank test for trend). Numbers in parentheses represent sample sizes.

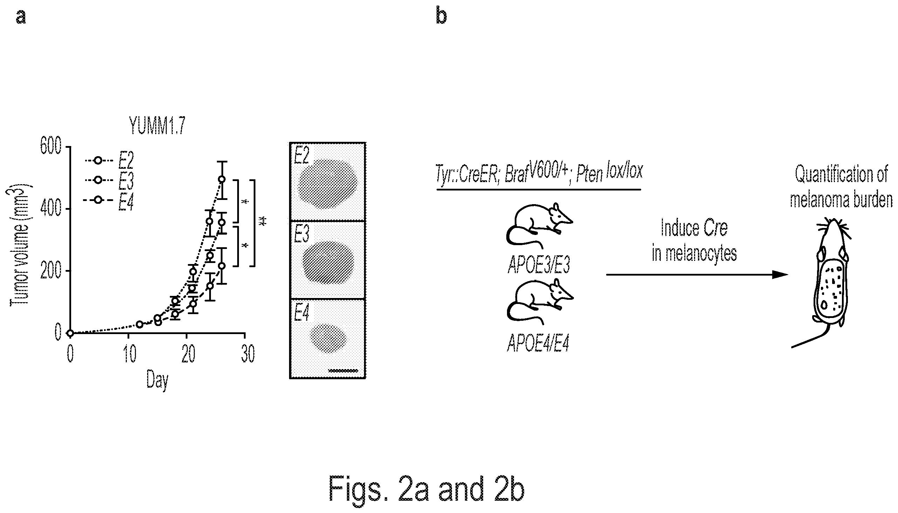

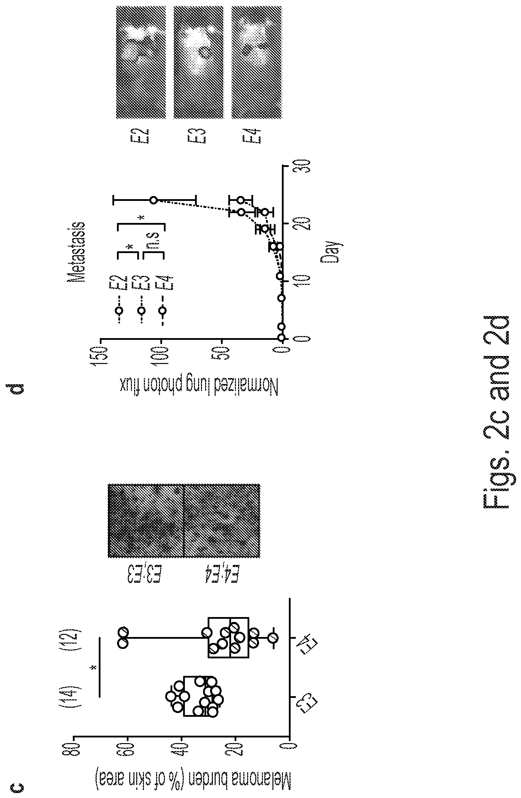

FIGs. 2a, 2b, 2c, and 2d are a set of diagrams showing that human APOE variants dictate tumor progression in mouse melanoma models. FIG. 2a shows that human APOE variants modulate progression of the transplantable *Braf-mutant* YUMM1.7 mouse melanoma model. Tumor volume of 100,000 YUMM1.7 cells injected into human APOE-targeted replacement mice (n = 9 - 11 per group, *p < 0.05, **p < 0.01, one-tailed t-test; data representative of two independent experiments). Representative tumors correspond to day 26 (scale bar, 5 mm). FIG. 2b shows a schematic of the experimental approach to assess the impact of human APOE alleles on genetically induced mouse melanoma progression. Human APOE3 and APOE4 knock-in mice were crossed to *Tyr::CreER; Braf*^{*V600E*/+}*;Pten*^{*lox*/*lox*} mice that develop genetically driven melanoma upon Tamoxifen-induced and melanocyte-specific expression of *Cre.* FIG. 2c shows that APOE4/E4 mice exhibit reduced tumor burden in comparison to APOE3/E3 mice in a genetically induced melanoma model. Melanoma burden measured as the percentage of the dorsal skin area of mice described in FIG. 2c on day 35 after melanoma induction (p = 0.01, one-tailed Mann-Whitney test). Images of representative skins are shown on the right. *p < 0.05. Box plots show median, first and third quartiles, and whiskers represent minimum and maximum. All data points are shown with each circle corresponding to one mouse. Numbers in parentheses indicate samples sizes. FIG 2d shows that that stromal APOE genotype also modulates metastatic progression of 100,000 B16F10-TR-shApoE cells injected intravenously (n = 10 per group; one-tailed Mann-Whitney test; representative of two independent experiments).

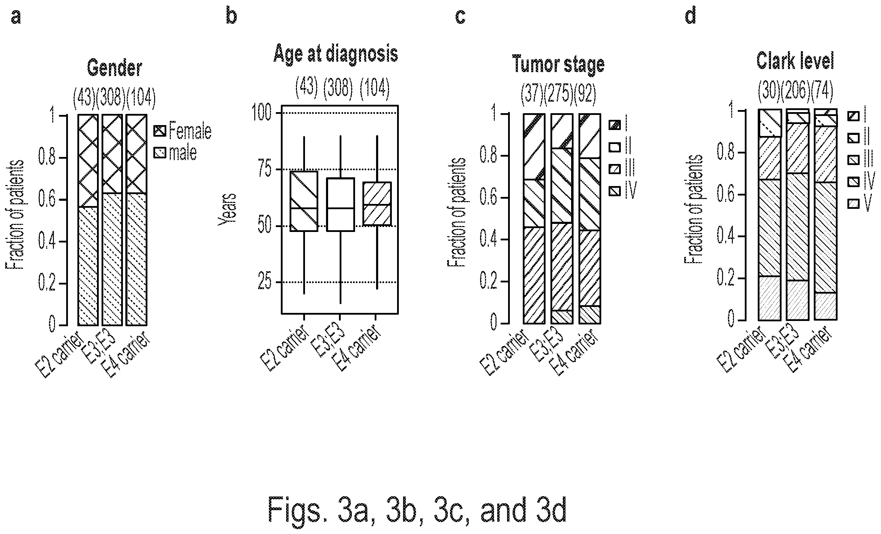

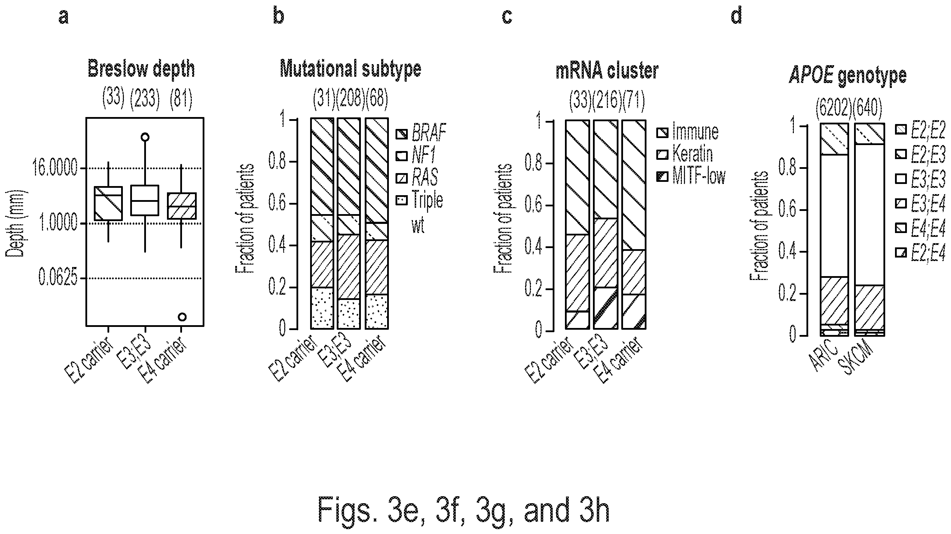

FIGs. 3a, 3b, 3c, 3d, 3e, 3f, 3g, and 3h are a set of diagrams showing bi-allelic APOE distribution and clinical characteristics of the TCGA- SKCM cohort. FIG. 3a shows that gender proportions were not significantly different between APOE carrier groups (P = 0.717, χ² test). FIG. 3b shows that age at diagnosis was not significantly different between APOE carrier groups (P = 0.999, ANOVA). FIG. 3c shows that the tumor stage at diagnosis was not significantly different between APOE carrier groups (P = 0.1703, χ² test). FIG. 3d shows that melanoma Clark level at diagnosis was not significantly different between APOE carrier groups (P = 0.6579, χ² test). FIG. 3e shows that Breslow depth was not significantly different between APOE carrier groups at diagnosis (P= 0.2814, Kruskal-Wallis rank-sum test). FIG. 3f shows the proportion of APOE carrier status across molecular subtypes of the TCGA-SKCM cohort. As indicated, APOE carrier status was not significantly associated with tumor mutational subtype (P= 0.8703, χ² test). FIG. 3g shows the proportion of APOE carrier status across transcriptional clusters of the TCGA-SKCM cohort. As indicated, APOE carrier status was not significantly associated with the transcriptomic cluster (P= 0.1176, χ² test). FIG. 3h shows the proportion of APOE genotypes in the Atherosclerosis Risk in Communities (ARIC) and the TCGA-SKCM cohorts. As indicated, the distribution of the bi-allelic APOE genotype was modestly skewed in the TCGA-SKCM cohort versus a healthy population (P = 0.0056, χ² test).

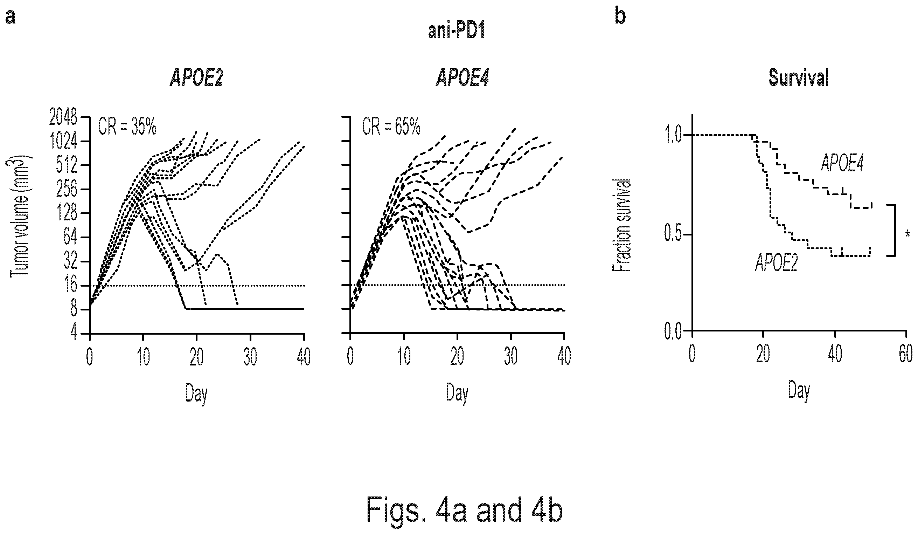

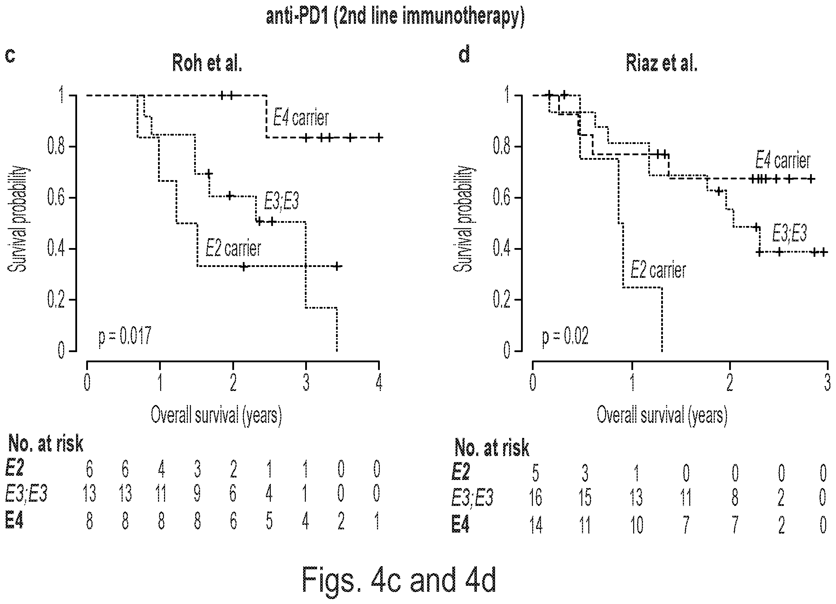

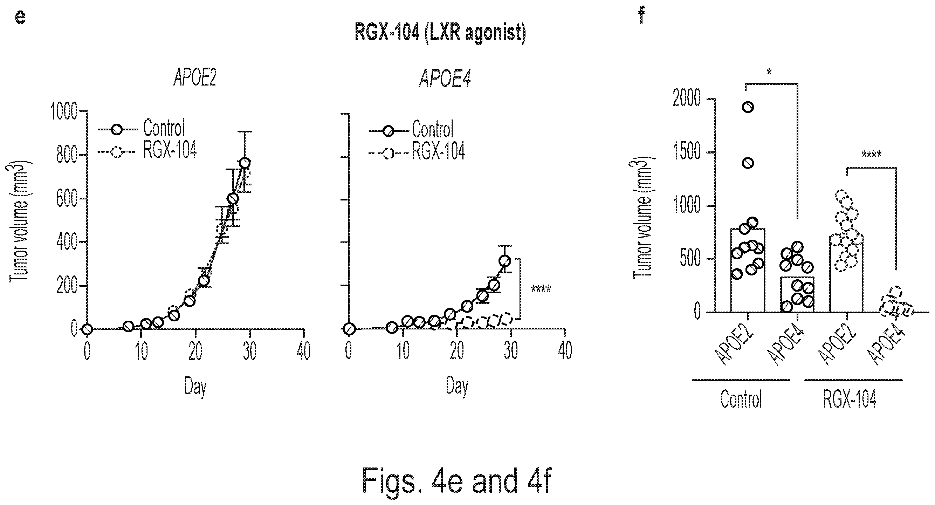

FIGs. 4a, 4b, 4c, 4d, 4e, and 4f are a set of diagrams showing APOE genotype modulates melanoma progression under immunotherapeutic regimens. FIG 4a shows tumor growth, and FIG 4b shows survival of human APOE knock-in mice injected with YUMMER1.7 tumors and treated with anti-PD1 antibody (n= 26 per group; P = 0.022, two-tailed log-rank test; data pooled from two independent experiments; CR = complete remission). FIGs 4c and 4d show survival of melanoma patients treated anti-PD1 immunotherapy after failing anti-CTLA4 treatment in the Roh *et al.* and Riaz *et al.* FIG 4d shows studies stratified by APOE carrier status (p values according to log-rank test). FIG 4e shows the effect of LXR-agonistic treatment on growth of YUMM1.7 tumors in human APOE knock-in mice (n ≥ 10 per group, two-tailed t-test; representative of two independent experiments). FIG 4f shows individual tumor volume on day 29 post injection from FIG 4e.

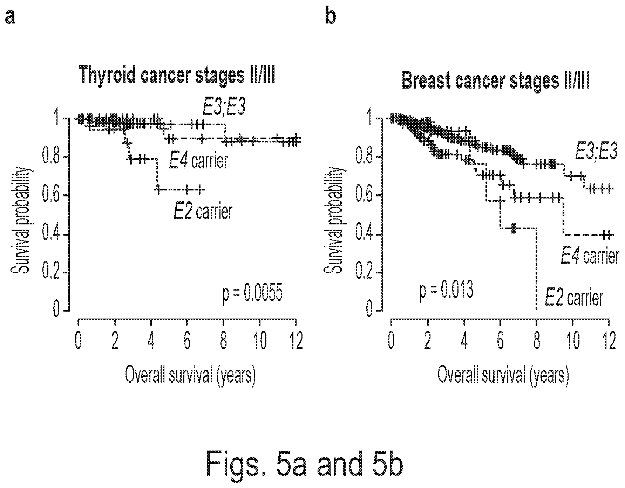

FIGs. 5a and 5b are a set of diagrams showing that APOE genotype modulates survival in thyroid and breast cancer. Survival of stage II/III thyroid (FIG. 5a) and breast (FIG. 5b) cancer patients from the TCGA cohorts stratified by APOE carrier status (log-rank test).

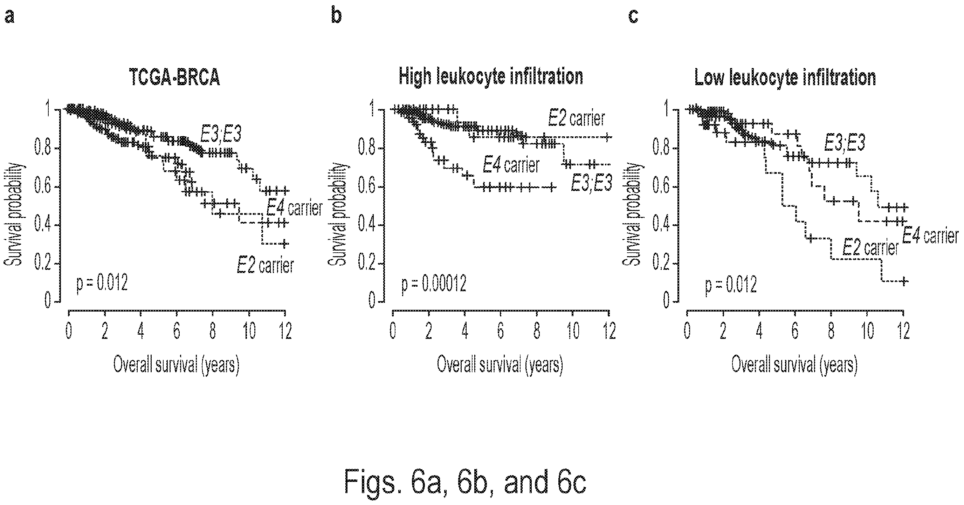

FIGs. 6a, 6b, and 6c are a set of diagrams showing that the impact of APOE genotype on breast cancer survival depends on tumor leukocyte infiltration. FIG. 6a shows survival of breast cancer patients in the TCGA cohort stratified by APOE genotype. FIGs. 6a, 6b, and 6c show survival of breast cancer patients from (FIG. 6a) stratified by high (FIG. 6b) versus low (FIG. 6c) leukocyte infiltration as assessed by RNA-seq deconvolution.

### DETAILED DESCRIPTION OF THE INVENTION

One of the most challenging features of human cancer is its unpredictable course after diagnosis. This disclosure provides methods and reagents for cancer prognosis, based, in part, on an unexpected discovery that in human melanoma common genetic germline polymorphisms of APOE, present in approximately 39% of Caucasians, predict cancer progression and survival outcomes. Analysis of the Cancer Genome Atlas (TCGA) revealed that carriers of the APOE2 variant experienced worse melanoma survival outcomes relative to those bearing the more common APOE3 variant. In contrast, carriers of the APOE4 variant exhibited improved melanoma survival relative to APOE3 homozygotes. The impact of these alleles on melanoma outcomes was dose-dependent, with APOE2 homozygotes experiencing the shortest and APOE4 homozygotes exhibiting the longest survival. Tumors grew more efficiently in human APOE2 than in APOE3 knock-in mice and least efficiently in APOE4 mice, suggesting APOE variants to causally regulate melanoma progression. Thus, the APOE4 and APOE2 germline variants reduce and increase, respectively, the likelihood of melanoma progression, an inversion of their risk modifying roles in Alzheimer's disease. The findings are consistent with the notion that germline genetic makeup can impact the trajectory of a future malignancy.

The secreted glycoprotein Apolipoprotein E (APOE) was previously found to suppress melanoma progression and metastasis through cancer cell autonomous and non-autonomous mechanisms. In humans, there are three prevalent APOE variants termed APOE2, APOE3, and APOE4, which differ in only two amino acids at positions 112 and 158 (FIG. 1a) (Chen, J. et al. PNAS 108, 14813-14818 (2011)). These variants are major genetic modifiers of risk for Alzheimer's and cardiovascular diseases (Strittmatter, W. J. et al. PNAS 90, 1977-1981 (1993); Corder, E. H. et al. Nat Genet 7, 180-184 (1994); Mahley, R. W. J Mol Med (Bed) 94, 739-746 (2016)), but their potential association with cancer outcome has so far remained inconclusive.

Given prior evidence supporting a role for APOE in melanoma progression, the association between germline APOE variant status and the clinical outcome of melanoma patients were investigated by determining APOE genotypes from normal tissue whole-exome sequencing data of 470 participants of the TCGA-SKCM study (FIG. 1b) (Network, T. C. G. A. (2015). Cell, 161(7), 1681-1696). In comparison to a control group with similar age and ethnic composition, homozygotes for the most common APOE3 variant (*i.e.,* non-E2/E4 carriers) were modestly overrepresented in the TCGA-SKCM cohort, indicating that neither APOE2 nor APOE4 carrier status predisposed for increased melanoma incidence (FIG. 1c). Strikingly, however, APOE carrier status was significantly associated with survival. E2 carriers, E3;E3 homozygotes, and E4 carriers have a median survival of 3.9, 5.5, and 10.1 years, respectively (FIG. 1d). There were no significant differences in potentially confounding characteristics such as gender, age, tumor stage, melanoma Clark level, Breslow depth, and mutational or transcriptomic subtype between APOE carrier groups at the time of diagnosis (FIGs. 3a-g). Remarkably, stratifying patients by bi-allelic APOE genotype revealed a gene dosage effects on survival (median survival of 3.6, 3.9, 5.8, 9.4 years and median survival not reached for E2;E2, E2;E3, E3;E3, E3;E4, and E4;E4, respectively) (FIG. 1f and FIG. 3h). Patients with an advanced local disease are at increased risk for metastatic progression and thus require careful consideration for systemic therapy. Because such therapies carry mortality and morbidity risks, they would be ideally administered to those at highest risk for progression. Thus, whether in the TCGA-SKCM cohort the association of APOE genotype with survival was maintained in patients with stage melanoma was further assessed. Indeed, APOE genotype stratified patient survival among this subgroup, suggesting that APOE genotype could be employed for risk stratifying these patients (FIG. 1e). Thus, APOE genotype was significantly associated with survival in melanoma in a gene-dosage-dependent fashion.

To assess whether APOE genotype merely correlated with or was causally involved in altering melanoma outcome, mice in which the endogenous murine APOE locus is replaced with one of the three human APOE variants were utilized. Using the Braf-mutant YUMM1.7 melanoma model we observed significantly slower tumor progression in E4;E4 and faster tumor progression in E2;E2 mice in comparison with E3;E3 mice consistent with APOE genotype causally impacting melanoma progression (FIG. 2a). With a combined allelic frequency of 91.6% in Caucasians, APOE3 and APOE4 are the most prevalent alleles (Farrer, L. A. etal. JAMA 278, 1349-1356 (1997)). Given their abundance, the impact of these APOE genotypes on a genetically initiated melanoma model, in which the tumor compartment also expresses the corresponding variant, was assessed. To this end, APOE3 and APOE4 mice to *Tyr:CreER; Braf*^{*V600E*/+}*;Pten*^{*lox*/*lox*} mice were crossed, in which melanoma formation can be initiated through Tamoxifen-induced and Cre-mediated excision of *Pten* in melanocytes (FIG. 2b). Consistent with the results in the transplantable melanoma models, mice with the E4;E4 genotype to exhibit reduced melanoma tumor burden in comparison to E3;E3 mice were observed (FIG. 2c). Additionally, the impact of APOE genotype on metastatic progression using the highly aggressive B 16F10-TR-shApoE model (depleted for endogenous mouse ApoE) were assessed. It was found that metastatic burden progressed significantly faster in APOE2 mice than in APOE3 and APOE4 mice (FIG. 2d). Therefore, human APOE alleles causally govern progression in syngeneic mouse melanoma models consistent with the clinical association data described above.

The results described herein reveal that genetic variants present in the germlines can predict and influence the progression of a common human cancer. In particular, common variants of APOE, a pleiotropic suppressor of metastatic melanoma phenotypes, exhibit differential effects on tumor progression. Thus, this disclosure provides methods for treating cancer based on the presence of APOE genotypes. Additionally, this disclosure provides methods for making medical and clinical decisions on neoadjuvant and adjuvant therapy, tumor surveillance, and the likelihood of disease progression based on the presence of APOE genotypes that are casually associated with tumor progression.

### I. METHODS OF TREATING CANCER AND CANCER PROGNOSIS

### a. Method of Treating Cancer

In one aspect, this disclosure provides a method of treating cancer in a subject having at least one ε4 allele from polymorphisms at rs7412 or rs429358 or ε3 alleles from polymorphisms at rs7412 and rs429358 in the *ApoE* gene on chromosome 19. The method comprises administering to the subject an effective amount of anti-cancer or anti-tumor agent, or a pharmaceutically acceptable salt thereof. In some embodiments, the anti-cancer or anti-tumor agent is an LXRβ agonist.

APOE is polymorphic with three major alleles/isoforms, as defined by two single nucleotide polymorphisms (SNPs), rs429358 and rs7412, known as ApoE-ε2, ApoE-ε3, and ApoE-ε4. The proteins produced by these genes are called ApoE2, ApoE3, and ApoE4. In some embodiments, APOE2 may include Cys112 and Cys158 residues, APOE3 may include Cys112 and Arg158 residues, and APOE4 may include Arg112 and Arg158 residues.

Although these allelic forms differ from each other by only one or two amino acids at positions 112 and 158, these differences alter APOE structure and function. The nucleotide sequences of human APOE2, APOE3, and APOE4 are shown in Table 1. In some embodiments, the particular APOE genotype may include an APOE gene having a sequence of SEQ ID NOs: 1-3.

**Table 1. Sequences of APOE variants**

| SEQ ID NO | SEQUENCES | OTHER INFORMATI ON |
|---|---|---|
| SEQ ID NO: 1 | ATGAAGGTTCTGTGGGCTGCGTTGCTGGTCACATT CCTGGCAGGATGCCAGGCCAAGGTGGAGCAAGCG GTGGAGACAGAGCCGGAGCCCGAGCTGCGCCAGC AGACCGAGTGGCAGAGCGGCCAGCGCTGGGAACT GGCACTGGGTCGCTTTTGGGATTACCTGCGCTGGG TGCAGACACTGTCTGAGCAGGTGCAGGAGGAGCTG CTCAGCTCCCAGGTCACCCAGGAACTGAGGGCGCT GATGGACGAGACCATGAAGGAGTTGAAGGCCTAC | APOE2 |
| | AAATCGGAACTGGAGGAACAACTGACCCCGGTGG CGGAGGAGACGCGGGCACGGCTGTCCAAGGAGCT GCAGGCGGCGCAGGCCCGGCTGGGCGCGGACATG GAGGACGTGTGCGGCCGCCTGGTGCAGTACCGCGG CGAGGTGCAGGCCATGCTCGGCCAGAGCACCGAG GAGCTGCGGGTGCGCCTCGCCTCCCACCTGCGCAA GCTGCGTAAGCGGCTCCTCCGCGATGCCGATGACC TGCAGAAGTGCCTGGCAGTGTACCAGGCCGGGGCC CGCGAGGGCGCCGAGCGCGGCCTCAGCGCCATCC GCGAGCGCCTGGGGCCCCTGGTGGAACAGGGCCG CGTGCGGGCCGCCACTGTGGGCTCCCTGGCCGGCC AGCCGCTACAGGAGCGGGCCCAGGCCTGGGGCGA GCGGCTGCGCGCGCGGATGGAGGAGATGGGCAGC CGGACCCGCGACCGCCTGGACGAGGTGAAGGAGC AGGTGGCGGAGGTGCGCGCCAAGCTGGAGGAGCA GGCCCAGCAGATACGCCTGCAGGCCGAGGCCTTCC AGGCCCGCCTCAAGAGCTGGTTCGAGCCCCTGGTG GAAGACATGCAGCGCCAGTGGGCCGGGCTGGTGG AGAAGGTGCAGGCTGCCGTGGGCACCAGCGCCGC CCCTGTGCCCAGCGACAATCAC | |
| SEQ ID NO: 2 | ATGAAGGTTCTGTGGGCTGCGTTGCTGGTCACATT CCTGGCAGGATGCCAGGCCAAGGTGGAGCAAGCG GTGGAGACAGAGCCGGAGCCCGAGCTGCGCCAGC AGACCGAGTGGCAGAGCGGCCAGCGCTGGGAACT GGCACTGGGTCGCTTTTGGGATTACCTGCGCTGGG TGCAGACACTGTCTGAGCAGGTGCAGGAGGAGCTG CTCAGCTCCCAGGTCACCCAGGAACTGAGGGCGCT GATGGACGAGACCATGAAGGAGTTGAAGGCCTAC AAATCGGAACTGGAGGAACAACTGACCCCGGTGG CGGAGGAGACGCGGGCACGGCTGTCCAAGGAGCT GCAGGCGGCGCAGGCCCGGCTGGGCGCGGACATG GAGGACGTGTGCGGCCGCCTGGTGCAGTACCGCGG CGAGGTGCAGGCCATGCTCGGCCAGAGCACCGAG | APOE3 |
| | GAGCTGCGGGTGCGCCTCGCCTCCCACCTGCGCAA GCTGCGTAAGCGGCTCCTCCGCGATGCCGATGACC TGCAGAAGCGCCTGGCAGTGTACCAGGCCGGGGCC CGCGAGGGCGCCGAGCGCGGCCTCAGCGCCATCC GCGAGCGCCTGGGGCCCCTGGTGGAACAGGGCCG CGTGCGGGCCGCCACTGTGGGCTCCCTGGCCGGCC AGCCGCTACAGGAGCGGGCCCAGGCCTGGGGCGA GCGGCTGCGCGCGCGGATGGAGGAGATGGGCAGC CGGACCCGCGACCGCCTGGACGAGGTGAAGGAGC AGGTGGCGGAGGTGCGCGCCAAGCTGGAGGAGCA GGCCCAGCAGATACGCCTGCAGGCCGAGGCCTTCC AGGCCCGCCTCAAGAGCTGGTTCGAGCCCCTGGTG GAAGACATGCAGCGCCAGTGGGCCGGGCTGGTGG AGAAGGTGCAGGCTGCCGTGGGCACCAGCGCCGC CCCTGTGCCCAGCGACAATCAC | |
| SEQ ID NO: 3 | ATGAAGGTTCTGTGGGCTGCGTTGCTGGTCACATT CCTGGCAGGATGCCAGGCCAAGGTGGAGCAAGCG GTGGAGACAGAGCCGGAGCCCGAGCTGCGCCAGC AGACCGAGTGGCAGAGCGGCCAGCGCTGGGAACT GGCACTGGGTCGCTTTTGGGATTACCTGCGCTGGG TGCAGACACTGTCTGAGCAGGTGCAGGAGGAGCTG CTCAGCTCCCAGGTCACCCAGGAACTGAGGGCGCT GATGGACGAGACCATGAAGGAGTTGAAGGCCTAC AAATCGGAACTGGAGGAACAACTGACCCCGGTGG CGGAGGAGACGCGGGCACGGCTGTCCAAGGAGCT GCAGGCGGCGCAGGCCCGGCTGGGCGCGGACATG GAGGACGTGCGCGGCCGCCTGGTGCAGTACCGCGG CGAGGTGCAGGCCATGCTCGGCCAGAGCACCGAG GAGCTGCGGGTGCGCCTCGCCTCCCACCTGCGCAA GCTGCGTAAGCGGCTCCTCCGCGATGCCGATGACC TGCAGAAGCGCCTGGCAGTGTACCAGGCCGGGGCC CGCGAGGGCGCCGAGCGCGGCCTCAGCGCCATCC GCGAGCGCCTGGGGCCCCTGGTGGAACAGGGCCG | APOE4 |
| | CGTGCGGGCCGCCACTGTGGGCTCCCTGGCCGGCC AGCCGCTACAGGAGCGGGCCCAGGCCTGGGGCGA GCGGCTGCGCGCGCGGATGGAGGAGATGGGCAGC CGGACCCGCGACCGCCTGGACGAGGTGAAGGAGC AGGTGGCGGAGGTGCGCGCCAAGCTGGAGGAGCA GGCCCAGCAGATACGCCTGCAGGCCGAGGCCTTCC AGGCCCGCCTCAAGAGCTGGTTCGAGCCCCTGGTG GAAGACATGCAGCGCCAGTGGGCCGGGCTGGTGG AGAAGGTGCAGGCTGCCGTGGGCACCAGCGCCGC CCCTGTGCCCAGCGACAATCAC | |

In many embodiments, the terms "subject" and "patient" are used interchangeably irrespective of whether the subject has or is currently undergoing any form of treatment. As used herein, the terms "subject" and "subjects" may refer to any vertebrate, including, but not limited to, a mammal (*e.g.,* cow, pig, camel, llama, horse, goat, rabbit, sheep, hamsters, guinea pig, cat, dog, rat, and mouse, a non-human primate (for example, a monkey, such as a cynomolgus monkey, chimpanzee, etc.) and a human). The subject may be a human or a non-human. In this context, a "normal," "control," or "reference" subject, patient or population is/are one(s) that exhibit(s) no detectable disease or disorder, respectively. In some embodiments, a "normal," "control," or "reference" subject has an APOE3 genotype.

In more exemplary aspects, the mammal is a human. As used herein, the expression "a subject in need thereof' or "a patient in need thereof' means a human or non-human mammal that exhibits one or more symptoms or indications of cancer or tumor, and/or who has been diagnosed with tumor or cancer, including a solid tumor and who needs treatment for the same. The expression includes subjects with primary, established, or recurrent tumor lesions. In specific embodiments, the expression includes human subjects that have and/or need treatment for a solid tumor. The expression also includes subjects with primary or metastatic tumors (advanced malignancies). In certain embodiments, the expression includes patients with a solid tumor that is resistant to or refractory to or is inadequately controlled by prior therapy *(e.g.,* surgery or treatment with an anti-cancer agent such as carboplatin or docetaxel). In certain embodiments, the expression includes patients with a tumor lesion that has been treated with one or more lines of prior therapy (*e.g.,* surgically removed), but which has subsequently recurred. In some embodiments, the expression includes subjects with a tumor or cancer who are not candidates for curative surgery or curative radiation, or for whom conventional anti-cancer therapy is inadvisable, for example, due to toxic side effects. In other embodiments, the expression includes subjects with a tumor lesion for which surgical removal is planned. In other embodiments, the expression includes subjects for whom the risk of recurrence is high due to a prior history of recurrence after surgery.

As used to describe the present invention, "cancer," "tumor," and "malignancy" all relate equivalently to hyperplasia of a tissue or organ. If the tissue is a part of the lymphatic or immune system, malignant cells may include non-solid tumors of circulating cells. Malignancies of other tissues or organs may produce solid tumors. The methods of the present invention may be used in the treatment of lymphatic cells, circulating immune cells, and solid tumors.

Cancers that can be treated include tumors that are not vascularized or are not substantially vascularized, as well as vascularized tumors. Cancers may comprise non-solid tumors (such as hematologic tumors, *e.g*., leukemias and lymphomas) or may comprise solid tumors. The types of cancers to be treated with the compositions of the present invention include, but are not limited to, carcinoma, blastoma and sarcoma, and certain leukemias or malignant lymphoid tumors, benign and malignant tumors and malignancies, *e*.*g*., sarcomas, carcinomas, and melanomas. Also included are adult tumors/cancers and pediatric tumors/cancers.

In some embodiments, the cancer is breast cancer, colon cancer, renal cell cancer, lung cancer, hepatocellular carcinoma, gastric cancer, ovarian cancer, pancreatic cancer, esophageal cancer, prostate cancer, sarcoma, bladder cancer, head and neck cancer, glioblastoma, diffuse large B-cell lymphoma, leukemia, or melanoma. In some embodiments, the cancer is melanoma, small cell lung cancer, or non-small cell lung cancer. In some embodiments, the cancer is metastatic and/or locally advanced. In some embodiments, the cancer is unresectable.

As used herein, the terms "treating," "treat," or the like, refer to alleviating or reducing the severity of at least one symptom or indication, eliminating the causation of symptoms either on a temporary or permanent basis, delaying or inhibiting tumor growth, reducing tumor cell load or tumor burden, promoting tumor regression, causing tumor shrinkage, necrosis and/or disappearance, preventing tumor recurrence, preventing or inhibiting metastasis, inhibiting metastatic tumor growth, eliminating the need for surgery, and/or increasing duration of survival of the subject.

The term "agent" is used herein to denote a chemical compound, a mixture of chemical compounds, a biological macromolecule (such as a nucleic acid, an antibody, a protein or portion thereof, *e.g.,* a peptide), or an extract made from biological materials such as bacteria, plants, fungi, or animal (particularly mammalian) cells or tissues. The activity of such agents may render it suitable as a "therapeutic agent," which is a biologically, physiologically, or pharmacologically active substance (or substances) that acts locally or systemically in a subject.

The terms "therapeutic agent," "therapeutic capable agent," or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder, or condition; and generally counteracting a disease, symptom, disorder or pathological condition.

As used herein, the term "pharmaceutically acceptable" refers to a material, such as a carrier or diluent, which does not abrogate the biological activity or properties of the composition, and is relatively non-toxic, *i.e.,* the material may be administered to an individual without causing undesirable biological effects or interacting in a deleterious manner with any of the components of the composition in which it is contained.

The term "pharmaceutically acceptable carrier" includes a pharmaceutically acceptable salt, pharmaceutically acceptable material, composition or carrier, such as a liquid or solid filler, diluent, excipient, solvent or encapsulating material, involved in carrying or transporting a compound(s) of the present invention within or to the subject such that it may perform its intended function. Typically, such compounds are carried or transported from one organ, or portion of the body, to another organ, or portion of the body. Each salt or carrier must be "acceptable" in the sense of being compatible with the other ingredients of the formulation, and not injurious to the subject. Some examples of materials that may serve as pharmaceutically acceptable carriers include: sugars, such as lactose, glucose, and sucrose; starches, such as corn starch and potato starch; cellulose, and its derivatives, such as sodium carboxymethyl cellulose, ethyl cellulose, and cellulose acetate; powdered tragacanth; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; diluent; granulating agent; lubricant; binder; disintegrating agent; wetting agent; emulsifier; coloring agent; release agent; coating agent; sweetening agent; flavoring agent; perfuming agent; preservative; antioxidant; plasticizer; gelling agent; thickener; hardener; setting agent; suspending agent; surfactant; humectant; carrier; stabilizer; and other non-toxic compatible substances employed in pharmaceutical formulations, or any combination thereof. As used herein, "pharmaceutically acceptable carrier" also includes any and all coatings, antibacterial and antifungal agents, and absorption delaying agents, and the like that are compatible with the activity of one or more components of the invention, and are physiologically acceptable to the subject. Supplementary active compounds may also be incorporated into the compositions.

The term "effective amount," "effective dose," or "effective dosage" is defined as an amount sufficient to achieve or at least partially achieve a desired effect. A "therapeutically effective amount" or "therapeutically effective dosage" of a drug or therapeutic agent is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A "prophylactically effective amount" or a "prophylactically effective dosage" of a drug/agent is an amount of the drug that, when administered alone or in combination with another therapeutic agent to a subject at risk of developing a disease or of suffering a recurrence of disease, inhibits the development or recurrence of the disease. The ability of a therapeutic or prophylactic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

In some embodiments, the effective amount comprises an amount effective to suppress metastatic colonization of the cancer.

In another aspect, this disclosure provides a method of treating cancer in a subject. The method comprises: (a) providing a subject identified as having at least one ε4 allele from polymorphisms at rs7412 or rs429358 or ε3 alleles from polymorphisms at rs7412 and rs429358 in the *ApoE* gene on chromosome 19; and (b) administering to the subject an effective amount of an anti-cancer or anti-tumor agent (*e.g.,* LXRβ agonist), or a pharmaceutically acceptable salt thereof.

In some embodiments, the method comprises: (a) providing a subject identified as having at least one ε4 allele from polymorphisms at rs7412 or rs429358 or ε3 alleles from polymorphisms at rs7412 and rs429358 in the *ApoE* gene on chromosome 19 and identified as likely to benefit from treatment with an anti-cancer or anti-tumor agent (*e.g.,* LXRβ agonist), or a pharmaceutically acceptable salt thereof, based on the presence of the at least one ε4 allele from polymorphisms at rs7412 or rs429358 or ε3 alleles from polymorphisms at rs7412 and rs429358; and (b) administering an effective amount of the anti-cancer or antitumor agent *(e.g.,* LXRβ agonist), or a pharmaceutically acceptable salt thereof, to the identified subject.

In some embodiments, the method comprises: (a) providing a subject identified as having at least one ε4 allele from polymorphisms at rs7412 or rs429358 or ε3 alleles from polymorphisms at rs7412 and rs429358 in the *ApoE* gene on chromosome 19 and identified as likely to benefit from treatment with an LXRβ agonist, or a pharmaceutically acceptable salt thereof, based on the presence of the at least one ε4 allele from polymorphisms at rs7412 or rs429358 or ε3 alleles from polymorphisms at rs7412 and rs429358; and (b) administering an effective amount of the LXRβ agonist, or a pharmaceutically acceptable salt thereof, to the identified subject.

In some embodiments, the method comprises (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with an anti-cancer or anti-tumor agent *(e.g.,* LXRβ agonist), or a pharmaceutically acceptable salt thereof; and (d) administering an effective amount of the anti-cancer or anti-tumor agent *(e.g.,* LXRβ agonist), or a pharmaceutically acceptable salt thereof, to the identified subject, whereby the cancer is treated.

In some embodiments, the method comprises (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with an LXRβ agonist, or a pharmaceutically acceptable salt thereof; and (d) administering an effective amount of the LXRβ agonist, or a pharmaceutically acceptable salt thereof, to the identified subject, whereby the cancer is treated.

"Sample," "test sample," and "patient sample" may be used interchangeably herein. The sample can be a sample of, serum, urine, plasma, amniotic fluid, cerebrospinal fluid, cells (*e.g.,* antibody-producing cells) or tissue. Such a sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art. The terms "sample" and "biological sample" as used herein generally refer to a biological material being tested for and/or suspected of containing an analyte of interest such as antibodies. The sample may be any tissue sample from the subject. The sample may comprise protein from the subject.

Any cell type, tissue, or bodily fluid may be utilized to obtain a sample. Such cell types, tissues, and fluid may include sections of tissues such as biopsy and autopsy samples, frozen sections taken for histologic purposes, blood (such as whole blood), plasma, serum, sputum, stool, tears, mucus, saliva, hair, skin, red blood cells, platelets, interstitial fluid, ocular lens fluid, cerebral spinal fluid, sweat, nasal fluid, synovial fluid, menses, amniotic fluid, semen, etc. Cell types and tissues may also include lymph fluid, ascetic fluid, gynecological fluid, urine, peritoneal fluid, cerebrospinal fluid, a fluid collected by vaginal rinsing, or a fluid collected by vaginal flushing. A tissue or cell type may be provided by removing a sample of cells from an animal, but can also be accomplished by using previously isolated cells *(e.g.,* isolated by another person, at another time, and/or for another purpose). Archival tissues, such as those having treatment or outcome history, may also be used. Protein purification may not be necessary.

Methods well known in the art for collecting, handling and processing urine, blood, serum, and plasma, and other body fluids, can be used in the practice of the present disclosure, for instance, when the antibodies provided herein are employed as immunodiagnostic reagents, and/or in an immunoassay kit. The test sample can comprise further moieties in addition to the analyte of interest, such as antibodies, antigens, haptens, hormones, drugs, enzymes, receptors, proteins, peptides, polypeptides, oligonucleotides or polynucleotides. For example, the sample can be a whole blood sample obtained from a subject. It can be necessary or desired that a test sample, particularly whole blood, be treated prior to immunoassay as described herein, *e.g.,* with a pretreatment reagent. Even in cases where pretreatment is not necessary, pretreatment optionally can be done for mere convenience *(e.g.,* as part of a regimen on a commercial platform). The sample may be used directly as obtained from the subject or following a pretreatment to modify a characteristic of the sample. Pretreatment may include extraction, concentration, inactivation of interfering components, and/or the addition of reagents.

The terms "increased," "increase" or "enhance" or "activate" are all used herein to generally mean an increase by a statically significant amount; for the avoidance of any doubt, the terms "increased," "increase" or "enhance" or "activate" means an increase of at least 10% as compared to a reference level, for example, an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

In another aspect, this disclosure also provides a method of identifying an individual having a cancer who may benefit from treatment with an LXRβ agonist, or a pharmaceutically acceptable salt thereof. The method comprises: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; and (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with an LXRβ agonist, or a pharmaceutically acceptable salt thereof, thereby identifying the individual having a cancer who may benefit from treatment with the LXRβ agonist, or a pharmaceutically acceptable salt thereof.

In some embodiments, the LXRβ agonist can be 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof. In some embodiments, the LXRβ agonist has a structure represented by:

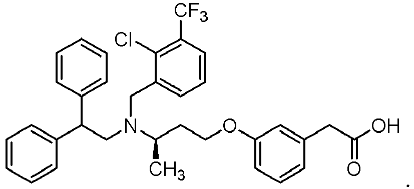

In another aspect, this disclosure further provides a method of treating cancer in a subject having at least one ε4 allele from polymorphisms at rs7412 or rs429358 or ε3 alleles from polymorphisms at rs7412 and rs429358 in the *ApoE* gene on chromosome 19. The method comprises administering an effective amount of a PD-1 inhibitor (e.g., nivolumab, pembrolizumab, pidilizumab, BMS 936559, and atezolizumab) or PD-L1 inhibitor (e.g., atezolizumab and durvalumab), or a pharmaceutically acceptable salt thereof, to the subject.

In another aspect, the method of treating cancer in a subject in need thereof comprises: (a) providing a subject identified as having at least one ε4 allele from polymorphisms at rs7412 or rs429358 or ε3 alleles from polymorphisms at rs7412 and rs429358 in the *ApoE* gene on chromosome 19; and (b) administering to the subject an effective amount of a PD-1 inhibitor (*e.g.,* nivolumab, pembrolizumab, pidilizumab, BMS 936559, and atezolizumab) or PD-L1 inhibitor (*e.g.,* atezolizumab and durvalumab), or a pharmaceutically acceptable salt thereof.

In some embodiments, the method comprises: (a) providing a subject identified as having at least one ε4 allele from polymorphisms at rs7412 or rs429358 or ε3 alleles from polymorphisms at rs7412 and rs429358 in the *ApoE* gene on chromosome 19 and identified as likely to benefit from treatment with a PD-1 inhibitor or PD-L1 inhibitor, or a pharmaceutically acceptable salt thereof, based on the presence of the at least one ε4 allele from polymorphisms at rs7412 or rs429358 or ε3 alleles from polymorphisms at rs7412 and rs429358; and (b) administering an effective amount of the PD-1 inhibitor or PD-L1 inhibitor to the identified subject.

In some embodiments, the method comprises: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with a PD-1 inhibitor or PD-L1 inhibitor, or a pharmaceutically acceptable salt thereof; and (d) administering an effective amount of the PD-1 inhibitor or PD-L1 inhibitor, or a pharmaceutically acceptable salt thereof, to the identified subject, whereby the cancer is treated.

In some embodiments, this disclosure provides a method of identifying an individual having a cancer who may benefit from treatment with a PD-1 inhibitor or PD-L1 inhibitor. The method comprising: (a) obtaining a sample containing nucleic acid from the subject; (b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; and (c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with a PD-1 inhibitor or PD-L1 inhibitor, or a pharmaceutically acceptable salt thereof, thereby identifying the individual having a cancer who may benefit from treatment with a PD-1 inhibitor or PD-L1 inhibitor, or a pharmaceutically acceptable salt thereof.

In some embodiments, the subject has been previously administered a CTLA-4 inhibitor, or a pharmaceutically acceptable salt thereof.

In some embodiments, the presence of at least one ε4 allele from polymorphisms at rs7412 or rs429358 or ε3 alleles from polymorphisms at rs7412 and rs429358 in the *ApoE* gene on chromosome 19 is determined by Polymerase Chain Reaction (PCR).

In some embodiments, the presence of at least one ε4 allele from polymorphisms at rs7412 or rs429358 or ε3 alleles from polymorphisms at rs7412 and rs429358 in the *ApoE* gene on chromosome 19 is determined by the expression level of APOE3 protein or APOE4 protein in the subject.

The level of APOE proteins may be measured by determining or estimating a protein level or mRNA level. Methods for determining or estimating a protein level or mRNA level are well known in the art. For example, the protein level (*e.g.,* protein expression level) of APOE proteins can be determined by SDS-PAGE, Western blot, or an immunoassay (*e.g.,* immunoblotting assay, immunoprecipitation assay). The mRNA level may be determined by RT-PCR.

Measuring the levels of APOE proteins can be performed by assaying the proteins themselves (by Western blotting, ELISA, RIA, and other techniques known to one skilled in the art), by assaying the mRNA encoding these proteins (such as quantitative PCR, Northern blotting, RNAse protection assay, RNA dot-blotting, and other techniques known to one skilled in the art), or by assaying the activity of the regulatory elements of the genes for APOE proteins. For example, the activity of regulatory elements can be assessed by reporter constructs consisting of DNA segments from the promoter, enhancer, and/or intronic elements coupled to cDNAs encoding reporters (such as luciferase, beta-galactosidase, green fluorescent protein, or other reporting genes that can be easily assayed). These reporter constructs can be transfected into cells, either stably, or transiently.

In some embodiments, the cancer is resistant, or has failed to respond to prior treatment with, to platinum-containing chemotherapy, a PD-1 inhibitor, a PD-L1 inhibitor, a CTLA-4 inhibitor, an antimitotic agent, a topoisomerase inhibitor, an antimetabolite, an angiogenesis inhibitor, a kinase inhibitor, and/or an alkylating agent. In some embodiments, the cancer progressed on or after treatment with platinum-containing chemotherapy, a PD-1 inhibitor, a PD-L1 inhibitor, an angiogenesis inhibitor, a kinase inhibitor, and/or an alkylating agent. In some embodiments, the cancer has been determined to be, or is predicted to be, resistant to a PD-1 inhibitor, a PD-L1 inhibitor, a CTLA-4 inhibitor, a topoisomerase inhibitor, an antimetabolite, an angiogenesis inhibitor, a kinase inhibitor, and/or an alkylating agent.

In some embodiments, the method further comprises administering an additional anti-cancer therapy to the subject. In some embodiments, the additional anti-cancer therapy comprises surgery, radiation, chemotherapy, and/or immunotherapy. In some embodiments, the additional anti-cancer therapy comprises chemotherapy and/or immunotherapy. In some embodiments, the chemotherapy comprises docetaxel, carboplatin or cisplatin, pemetrexed, and/or pembrolizumab.

In some embodiments, the method further comprises administering to the subject an effective amount of folic acid, vitamin B12, corticosteroids, a statin, an anti-emetic agent, an anti-diarrheal agent, an appetite stimulant, a general stimulant, a bisphosphonate, a gonadotrophin-releasing hormone agonist, growth factors, and/or an LHRH agonist.

In some embodiments, the subject has ε3 alleles from polymorphisms at rs7412 and rs429358 in the *ApoE* gene. In some embodiments, the subject has at least one ε4 allele from polymorphisms at rs7412 or rs429358 in the *ApoE* gene. In some embodiments, the subject has ε4 alleles from polymorphisms at rs7412 and rs429358, an ε3 allele and an ε4 allele from polymorphisms at rs7412 and rs429358, or an ε2 and an ε4 allele from polymorphisms at rs7412 and rs429358 in the *ApoE* gene. In some embodiments, the subject has ε4 alleles from polymorphisms at rs7412 and rs429358 in the *ApoE* gene.

In yet another aspect, this disclosure further provides a method of treating cancer in a subject having at least one ε2 allele from polymorphisms at rs7412 or rs429358 in the *ApoE* gene on chromosome 19. The method comprises administering an aggressive treatment to the subject.

In another aspect, this disclosure provides a method of treating cancer in a subject in need thereof. The method comprises: (a) providing a subject identified as having at least one ε2 allele from polymorphisms at rs7412 or rs429358 in the *ApoE* gene on chromosome 19; and (b) administering to the subject an aggressive treatment.

In some embodiments, the method comprises: (a) providing a subject identified as having at least one ε2 allele from polymorphisms at rs7412 or rs429358 in the *ApoE* gene on chromosome 19 and identified as likely to benefit from an aggressive treatment based on the presence of the at least one ε2 allele from polymorphisms at rs7412 and rs429358; and (b) administering an aggressive treatment to the identified subject.

In some embodiments, the method comprises: (a) obtaining a sample containing nucleic acid from the subject; (b) performing a genotyping assay on the sample and detecting the presence of at least one ε2 allele from polymorphisms at rs7412 or rs429358 in the *ApoE* gene on chromosome 19; (c) identifying the subject having the at least one ε2 allele from polymorphisms at rs7412 or rs429358 in the *ApoE* gene on chromosome 19 as having an increased likelihood of benefiting from an aggressive treatment; and (d) administering an aggressive treatment to the identified subject, whereby the cancer is treated.

In another aspect, this disclosure provides a method of identifying an individual having a cancer who may benefit from an aggressive treatment. The method comprises: (a) obtaining a sample containing nucleic acid from the subject; (b) performing a genotyping assay on the sample and detecting the presence of at least one ε2 allele from polymorphisms at rs7412 or rs429358 in the *ApoE* gene on chromosome 19; and (c) identifying the subject having the at least one ε2 allele from polymorphisms at rs7412 or rs42935 8 in the *ApoE* gene on chromosome 19 as having an increased likelihood of benefiting from an aggressive treatment, thereby identifying the individual having a cancer who may benefit from an aggressive treatment.

In some embodiments, the presence of at least one ε2 allele from polymorphisms at rs7412 or rs429358 in the *ApoE* gene on chromosome 19 is determined by Polymerase Chain Reaction (PCR). In some embodiments, the presence of at least one ε2 allele from polymorphisms at rs7412 or rs429358 in the *ApoE* gene on chromosome 19 is determined by the expression level of APOE2 protein in the subject.

In some embodiments, the subject has ε2 alleles from polymorphisms at rs7412 and rs429358 or an ε2 allele and an ε3 allele from polymorphisms at rs7412 and rs429358 in the *ApoE* gene on chromosome 19.

In some embodiments, the aggressive treatment comprises administering 2-[3-[(3R)-3-[[2-chloro-3-(trifluoromethyl)phenyl]methyl-(2,2-diphenylethyl)amino]butoxy]phenyl]acetic acid, or a pharmaceutically acceptable salt thereof and an immunotherapy in amounts that together are effective.

In some embodiments, the aggressive treatment further comprises performing surgery on the subject. In some embodiments, the 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof is administered prior to the surgery. In some embodiments, the 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof is administered after the surgery.

In some embodiments, the immunotherapy is a PD-1 inhibitor, a PD-L1 inhibitor, or a CTLA-4 inhibitor.

In some embodiments, the additional therapeutic agent may be administered to the subject before, after, or concurrently with the second therapeutic agent. The anti-cancer or anti-tumor agent *(e.g.,* LXRβ agonist, PD-1 inhibitor, or PD-L1 inhibitor), or a pharmaceutical composition thereof, can be administered as a single dose or more commonly can be administered on multiple occasions. Intervals between single dosages can be, for example, weekly, monthly, every three months or yearly. Intervals can also be irregular as indicated by measuring blood levels of anti-cancer or anti-tumor agent *(e.g.,* LXRβ agonist, PD-1 inhibitor, or PD-L1 inhibitor) to the patient.

The anti-cancer or anti-tumor agent *(e.g.,* LXRβ agonist, PD-1 inhibitor, or PD-L1 inhibitor), or a pharmaceutical composition thereof, can be administered via one or more routes of administration using one or more of a variety of methods known in the art. As will be appreciated by the skilled artisan, the route and/or mode of administration will vary depending upon the desired results. For example, the routes of administration for the anti-cancer or anti-tumor agent include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion. Alternatively, the anti-cancer or anti-tumor agent can be administered via a non-parenteral route, such as a topical, epidermal or mucosal route of administration, for example, intranasally, orally, vaginally, rectally, sublingually or topically.

In some embodiments, the anti-cancer or anti-agent, or a pharmaceutical composition thereof, can be administered intratumorally, intravenously, subcutaneously, intraosseously, orally, transdermally, in sustained release, in controlled release, in delayed release, as a suppository, or sublingually.

Dosage regimens are adjusted to provide the optimum desired response *(e.g.,* a therapeutic response). For example, a single bolus may be administered, several divided doses may be administered over time, or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active ingredient calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active ingredient for the treatment of sensitivity in individuals.

For administration of the anti-cancer or anti-tumor agent, the dosage ranges from about 0.0001 to 100 mg/kg, and more usually 0.01 to 5 mg/kg, of the host body weight. An exemplary treatment regime entails administration once per week, once every two weeks, once every three weeks, once every four weeks, once a month, once every 3 months or once every three to 6 months.

Alternatively, the anti-cancer or anti-tumor agent can be administered as a sustained release formulation, in which case less frequent administration is required. Dosage and frequency vary depending on the half-life of the anti-cancer or anti-tumor agent in the patient. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of the disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular active ingredient being employed, the duration of the treatment, other drugs, compounds and/or materials used in combination with the particular compositions employed, the age, sex, weight, condition, general health and prior medical history of the patient being treated, and like factors well known in the medical arts.

In some embodiments, the anti-cancer or anti-tumor agent *(e.g.,* LXRP agonist, PD-1 inhibitor, or PD-L1 inhibitor), or a pharmaceutical composition thereof, can be administered in combination therapy, *i.e.,* co-administered with additional therapeutic agents. For example, the combination therapy can include an LXRβ agonist, a PD-1 inhibitor, or PD-L1 inhibitor combined with at least one additional therapeutic agent, such as another anti-cancer or anti-tumor agent, including chemotherapeutic agents and immunotherapeutic agents, as provided above.

A "chemotherapeutic agent" is a chemical compound useful in the treatment of cancer. Examples of chemotherapeutic agents include alkylating agents such as thiotepa and cyclophosphamide (CYTOXANTM); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics *(e.g.* calicheamicin, see, *e.g.,* Agnew Chem. Intl. Ed. Engl. 33:183-186 (1994); dynemicin, including dynemicin A; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK^{®}.; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e.g.* paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

An "immunotherapeutic agent" is a biological agent useful in the treatment of cancer. Examples of immunotherapeutic agents include atezolizumab, avelumab, blinatumomab, daratumumab, cemiplimab, durvalumab, elotuzumab, laherparepvec, ipilimumab, nivolumab, obinutuzumab, ofatumumab, pembrolizumab, and talimogene.

In some embodiments, the subject has been administered an immune checkpoint inhibitor or an LXR agonist *(e.g.,* RGX-104). Examples of immune checkpoint inhibitors may include antibodies targeting CTLA-4, PD-1, PD-L1, PD-L2, killer immunoglobulin receptor (KIR), LAG3, B7-H3, B7-H4, TIM3, A2aR, CD40L, CD27, OX40, 4-IBB, TCR, BTLA, ICOS, CD28, CD80, CD86, ICOS-L, B7-H4, HVEM, 4-1BBL, OX40L, CD70, CD40, or GALS. In some embodiments, the immune checkpoint inhibitor is an anti-PD-1 antibody.

As used herein, the term "co-administration" or "co-administered" refers to the administration of at least two agent(s) or therapies to a subject. In some embodiments, the co-administration of two or more agents/therapies is concurrent. In other embodiments, a first agent/therapy is administered prior to a second agent/therapy. Those of skill in the art understand that the formulations and/or routes of administration of the various agents/therapies used may vary.

### b. Method of Cancer Prognosis

This disclosure also provides a method for assessing the prognosis of a subject that has or is at risk of having a cancer. The method includes (i) obtaining a sample from the subject; (ii) determining the presence of a particular APOE genotype in the sample; and (iii) determining a prognostic outcome of the subject based on the presence of the particular APOE genotype, such as APOE2, APOE3, or APOE4 genotype. In some embodiments, the method additionally includes administrating to the subject a drug for treating the cancer based on the presence of the particular APOE genotype to achieve a better prognostic outcome than the determined prognostic outcome.

In some embodiments, the sample may include a sample of cells, tissues, or bodily fluid from the subject, such as a tumor tissue or a blood sample. The presence of the particular APOE genotype in the sample can be determined by PCR. PCR primers are preferably chosen based on a known sequence of the genome that will result in amplification of specific fragments of genomic DNA. Computer programs that are well known in the art are useful in the design of primers with the required specificity and optimal amplification properties, such as Oligo version 5.0 (National Biosciences). In some embodiments, the PCR primer includes a sequence of SED ID NOs: 4-16.

In some embodiments, determining the presence of the particular APOE genotype includes determining the presence of a particular APOE variant protein in the sample. Determining the presence of the particular APOE variant protein in the sample can be performed using an antibody or fragment thereof that binds specifically to the particular APOE variant protein. Such APOE variant protein-specific antibodies may include, without limitation, APOE2 specific antibody: clone F48.1 (Research Diagnostics, NJ, USA), APOE4 specific antibody: clone 4E4 (Novus Biologicals, cat# NBP1-49529)Various assays can be used to determine the presence of the particular APOE variant protein using an antibody or fragment thereof, such as electrochemiluminescence assay, enhanced chemiluminescence assay, enzyme-linked immunosorbent assay (ELISA), and co-immunoprecipitation (co-IP).

Genotyping APOE polymorphism in a biological sample, such as tumor tissue samples or peripheral blood samples, can provide an early indication of recovery or lack thereof. For example, the presence of the APOE2 genotype is indicative of a poor prognosis for the subject, whereas the presence of the APOE4 genotype is indicative of a good prognosis for the subject. The prognosis of disease course includes a risk for metastasis, recurrence, and relapse. A good prognosis is indicative of longer survival rates relative to subjects with a poor prognosis.

The prognostic methods described herein provide information useful in prognostication, identifying progression of and management of conditions that are characterized by uncontrolled, autonomous cell growth. For example, the information more specifically assists the clinician in designing chemotherapeutic or other treatment regimens to eradicate such conditions from the body of an afflicted subject, such as a human. For example, the methods can be used to determine whether a subject is suitable to be administered with an agent *(e.g.,* an agonist, antagonist, peptidomimetic, protein, peptide, nucleic acid, small molecule, or other drug candidate) to treat a disorder associated with uncontrolled, autonomous cell growth.

The term "prognosis" as used herein refers to a prediction of the probable course and outcome of a clinical condition or disease. A prognosis is usually made by evaluating factors or symptoms of a disease that are indicative of a favorable or unfavorable course or outcome of the disease. The phrase "assessing the prognosis" or "determining the prognosis" as used herein refers to the process by which the skilled artisan can predict the course or outcome of a condition in a patient. The term "prognosis" does not refer to the ability to predict the course or outcome of a condition with 100% accuracy instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition.

The terms "good prognosis," "favorable prognosis," and "positive prognosis," or "poor prognosis," "unfavorable prognosis," and "negative prognosis" as used herein are relative terms for the prediction of the probable course and/or likely outcome of a condition or a disease. A good, favorable or positive prognosis predicts a better outcome for a condition than a poor, unfavorable or negative prognosis. In a general sense, a "good prognosis," "favorable prognosis," and "positive prognosis," is an outcome that is relatively better than many other possible prognoses that could be associated with a particular condition, whereas a "poor prognosis," "unfavorable prognosis," and "negative prognosis" predicts an outcome that is relatively worse than many other possible prognoses that could be associated with a particular condition. Typical examples of a good, favorable or positive prognosis include a better than average cure rate, a lower propensity for metastasis, a longer than expected life expectancy, differentiation of a benign process from a cancerous process, and the like. For example, a positive prognosis is one where a patient has a 50% probability of being cured of a particular cancer after treatment, while the average patient with the same cancer has only a 25% probability of being cured. In some embodiments, a "good prognosis," "favorable prognosis," and "positive prognosis" of an APOE4 carrier is relative to the prognostic outcome of an APOE3 carrier (considered the "neutral" APOE genotype). Similarly, a "poor prognosis," "unfavorable prognosis," and "negative prognosis" of an APOE2 carrier is relative to the prognostic outcome of an APOE3 carrier.

The prognostic methods described herein can be used to determine whether a subject is suitable for or needs a treatment, *e.g.,* surgery or administering with a therapeutic agent (such as an agonist, antagonist, peptidomimetic, protein, peptide, nucleic acid, small molecule, or other drug). For example, such methods can be used to determine whether a subject can be administered with an antibody or other therapy *(e.g.,* surgery such as amputation). Accordingly, certain methods described herein optionally further comprise treating the subject (*e.g.,* with one or more pharmaceutical compositions for a period of time or by surgery) if a subject receives a poor prognosis.

In some embodiments, the subject receiving the prognostic analysis has a cancer or is at risk of having a cancer. The cancer can be any one of oral cancer, oropharyngeal cancer, nasopharyngeal cancer, respiratory cancer, urogenital cancer, gastrointestinal cancer, central or peripheral nervous system tissue cancer, an endocrine or neuroendocrine cancer or hematopoietic cancer, glioma, sarcoma, carcinoma, lymphoma, melanoma, fibroma, meningioma, brain cancer, oropharyngeal cancer, nasopharyngeal cancer, renal cancer, biliary cancer, pheochromocytoma, pancreatic islet cell cancer, Li-Fraumeni tumors, thyroid cancer, parathyroid cancer, pituitary tumors, adrenal gland tumors, osteogenic sarcoma tumors, multiple neuroendocrine type I and type II tumors, breast cancer, lung cancer, head and neck cancer, prostate cancer, esophageal cancer, tracheal cancer, liver cancer, bladder cancer, stomach cancer, pancreatic cancer, ovarian cancer, uterine cancer, cervical cancer, testicular cancer, colon cancer, rectal cancer, and skin cancer.

### II. KITS AND ARRAYS FOR CANCER PROGNOSIS

This disclosure also provides kits and arrays embodying the above-described methods. Such kits can be prepared from readily available materials and reagents and can come in a variety of embodiments.

In some embodiments, the kit includes a probe having a nucleic acid sequence complementary to the nucleic acid sequence of APOE2, APOE3, or APOE4. Such a probe can hybridize to the nucleic acid sequence of APOE2, APOE3, or APOE4 under a stringent condition. In some embodiments, the kit includes labeled probes having sequences that are complementary to APOE2, APOE3, and APOE4, respectively. In some embodiments, the nucleic acid sequences of APOE2, APOE3, and APOE4 comprise the sequences of SEQ ID NOs: 1-3, respectively. In some embodiments, the nucleic acid sequence of the probe is 15-200 bases in length. In some embodiments, the probe is linked with a detection label. The detection label can be a fluorophore, a dye, a radiolabel, an enzyme, a biotin, or an antibody (or fragment thereof).

In some embodiments, the kit also includes primers configured to specifically bind sequences flanking a genomic region to allow PCR amplification of at least one of APOE2, APOE3, and APOE4 harboring the genomic region. In some embodiments, the primer may have a sequence of SED ID NOs: 4-16.

The kit can comprise a calibrator or control, *e.g*., purified, and optionally lyophilized, antigens, antibodies or combinations thereof. The kit can include at least one container (*e.g.,* tube, microtiter plates or strips, which can be already coated with a suitable antigen or antibody) for conducting the assay, and/or a buffer, such as an assay buffer or a wash buffer, either one of which can be provided as a concentrated solution, a substrate solution for the detectable label *(e.g.,* an enzymatic label), or a stop solution. Preferably, the kit comprises all components, *i.e.,* reagents, standards, buffers, diluents, etc., which are necessary to perform the assay. The antibodies, calibrators and/or controls can be provided in separate containers or pre-dispensed into an appropriate assay format, for example, into microtiter plates.

Optionally, the kit includes quality control components (for example, sensitivity panels, calibrators, and positive controls). Preparation of quality control reagents is well-known in the art and is described on insert sheets for a variety of diagnostic products. Sensitivity panel members optionally are used to establish assay performance characteristics, and further optionally are useful indicators of the integrity of the kit reagents, and the standardization of assays.

The kit can also optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a test sample (*e.g.,* pretreatment reagents), also can be included in the kit. The kit can additionally include one or more other controls. One or more of the components of the kit can be lyophilized, in which case the kit can further comprise reagents suitable for the reconstitution of the lyophilized components.

The various components of the kit optionally are provided in suitable containers as necessary, *e.g.,* a microtiter plate. The kit can further include containers for holding or storing a sample *(e.g.,* a container or cartridge for a urine sample). Where appropriate, the kit optionally also can contain reaction vessels, mixing vessels, and other components that facilitate the preparation of reagents or the test sample. The kit can also include one or more instrument for assisting with obtaining a test sample, such as a syringe, pipette, forceps, measured spoon, or the like.

If desired, the kit can contain a solid phase, such as a magnetic particle, bead, test tube, microtiter plate, cuvette, membrane, scaffolding molecule, film, filter paper, a quartz crystal, disc or chip. The kit may also include a detectable label that can be or is conjugated to an antigen or an antibody. The detectable label can, for example, be a direct label, which may be an enzyme, oligonucleotide, nanoparticle chemiluminophore, fluorophore, fluorescence quencher, chemiluminescence quencher, or biotin. Kits may optionally include any additional reagents needed for detecting the label.

In some examples, the kits can contain one or more of additional anti-cancer therapeutic agents, such as one or more chemotherapeutic or immunotherapeutic agents mentioned herein or known in the art. Such kits can include commercial packaging and/or printed information about the antibodies, anti-bacterium therapeutic agents and methods of their use.

Kits can contain instructions for their use. Instructions included in kits can be affixed to packaging material or can be included as a package insert. While the instructions are typically written or printed materials, they are not limited to such. Any medium capable of storing such instructions and communicating them to an end user is contemplated by this disclosure. Such media include, but are not limited to, electronic storage media *(e.g.,* magnetic discs, tapes, cartridges, chips), optical media (e.g., CD ROM), and the like. As used herein, the term "instructions" can include the address of an internet site that provides the instructions. The instructions also can include instructions for generating a standard curve or a reference standard for purposes of quantifying a target.

Also provided in this disclosure is a biochip or array. The biochip/array may contain a solid or semi-solid substrate having an attached probe or plurality of probes described herein. The probes may be capable of hybridizing to a target sequence under stringent hybridization conditions. The probes may be attached at the spatially defined address on the substrate. More than one probe per target sequence may be used, with either overlapping probes or probes to different sections of a particular target sequence. The probes may be capable of hybridizing to target sequences associated with a single disorder appreciated by those in the art. The probes may either be synthesized first, with subsequent attachment to the biochip or may be directly synthesized on the biochip.

In another aspect, this disclosure provides an array comprising a support having a plurality of unique locations. The array may include at least a nucleic acid or a probe having a sequence complementary to a nucleic acid sequence of APOE2, APOE3, or APOE4. In some embodiments, the nucleic acid sequence is one of SEQ ID NOs: 1-3.

The solid substrate can be a material that may be modified to contain discrete individual sites appropriate for the attachment or association of the probes and is amenable to at least one detection method. Examples of such substrates include glass and modified or functionalized glass, plastics (including acrylics, polystyrene, and copolymers of styrene and other materials, polypropylene, polyethylene, polybutylene, polyurethanes, TEFLON, etc.), polysaccharides, nylon or nitrocellulose, resins, silica or silica-based materials including silicon and modified silicon, carbon, metals, inorganic glasses, and plastics. The substrates may allow optical detection without appreciably fluorescing.

The substrate can be planar, although other configurations of substrates may be used as well. For example, probes may be placed on the inside surface of a tube, for flow-through sample analysis to minimize sample volume. Similarly, the substrate may be flexible, such as flexible foam, including closed-cell foams made of particular plastics.

The array/biochip and the probe may be derivatized with chemical functional groups for subsequent attachment of the two. For example, the biochip may be derivatized with a chemical functional group including, but not limited to, amino groups, carboxyl groups, oxo groups or thiol groups. Using these functional groups, the probes may be attached using functional groups on the probes either directly or indirectly using a linker. The probes may be attached to the solid support by either the 5' terminus, 3' terminus, or via an internal nucleotide. The probe may also be attached to the solid support non-covalently. For example, biotinylated oligonucleotides can be made, which may bind to surfaces covalently coated with streptavidin, resulting in attachment. Alternatively, probes may be synthesized on the surface using techniques such as photopolymerization and photolithography. Detailed discussion of methods for linking nucleic acids to a support substrate can be found in, *e.g.,* U.S. Patent Nos. 5837832, 6087112, 5215882, 5707807, 5807522, 5958342, 5994076, 6004755, 6048695, 6060240, 6090556, and 6040138.

### III. DEFINITIONS

To aid in understanding the detailed description of the compositions and methods according to the disclosure, a few express definitions are provided to facilitate an unambiguous disclosure of the various aspects of the disclosure. Unless otherwise defined, all technical and scientific terms used herein have the same meaning as commonly understood by one of ordinary skill in the art to which this disclosure belongs.

The term "allele" as used herein refers to one or more alternative forms of a gene occupying a given locus on a chromosome.

The term "gene" as used herein refers to a natural (*e.g.,* genomic) or synthetic gene comprising transcriptional and/or translational regulatory sequences and/or a coding region and/or non-translated sequences (*e.g.,* introns, 5'- and 3'-untranslated sequences). The coding region of a gene may be a nucleotide sequence coding for an amino acid sequence or a functional RNA, such as tRNA, rRNA, catalytic RNA, siRNA, miRNA, or antisense RNA. A gene may also be an mRNA or cDNA corresponding to the coding regions (*e.g.,* exons and miRNA) optionally comprising 5'- or 3'-untranslated sequences linked thereto. A gene may also be an amplified nucleic acid molecule produced *in vitro* comprising all or a part of the coding region and/or 5'- or 3'-untranslated sequences linked thereto. The term also includes pseudogenes, which are dysfunctional relatives of known genes that have lost their protein-coding ability or are otherwise no longer expressed in a cell.

"Nucleic acid" or "oligonucleotide" or "polynucleotide" as used herein refers to at least two nucleotides covalently linked together. The depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids may be single-stranded or double-stranded or may contain portions of both double-stranded and single-stranded sequence. The nucleic acid may be DNA, both genomic and cDNA, RNA, or a hybrid, where the nucleic acid may contain combinations of deoxyribo- and ribo-nucleotides, and combinations of bases including uracil, adenine, thymine, cytosine, guanine, inosine, xanthine hypoxanthine, isocytosine, and isoguanine. Nucleic acids may be obtained by chemical synthesis methods or by recombinant methods.

"Polypeptide" is used in its conventional meaning, *i.e.,* as a sequence of amino acids. The polypeptides are not limited to a specific length of the product. Peptides, polypeptides, and proteins are included within the definition of polypeptide, and such terms can be used interchangeably herein unless specifically indicated otherwise. This term also includes post-expression modifications of the polypeptide, for example, glycosylations, acetylations, phosphorylations and the like, as well as other modifications known in the art, both naturally occurring and non-naturally occurring. A polypeptide can be an entire protein or a subsequence thereof.

The term "primer" refers to any nucleic acid that is capable of hybridizing at its 3' end to a complementary nucleic acid molecule, and that provides a free 3' hydroxyl terminus which can be extended by a nucleic acid polymerase. As used herein, amplification primers are a pair of nucleic acid molecules that can anneal to 5' or 3' regions of a gene (plus and minus strands, respectively, or vice-versa) and contain a short region in between. Under appropriate conditions and with appropriate reagents, such primers permit the amplification of a nucleic acid molecule having the nucleotide sequence flanked by the primers. For in situ methods, a cell or tissue sample can be prepared and immobilized on a support, such as a glass slide, and then contacted with a probe that can hybridize to RNA. Alternative methods for amplifying nucleic acids corresponding to expressed RNA samples include those described in, *e.g.,* U.S. Patent No. 7,897,750.

The term "probe" as used herein refers to an oligonucleotide capable of binding to a target nucleic acid of complementary sequence through one or more types of chemical bonds, usually through complementary base pairing, usually through hydrogen bond formation. Probes may bind target sequences lacking complete complementarity with the probe sequence depending upon the stringency of the hybridization conditions. There may be any number of base pair mismatches which will interfere with hybridization between the target sequence and the single-stranded nucleic acids described herein. However, if the number of mutations is so great that no hybridization can occur under even the least stringent of hybridization conditions, the sequence is not a complementary target sequence. A probe may be single-stranded or partially single and partially double-stranded. The strandedness of the probe is dictated by the structure, composition, and properties of the target sequence. Probes may be directly labeled or indirectly labeled such as with biotin to which a streptavidin complex may later bind.

"Complement" or "complementary" as used herein to refer to a nucleic acid may mean Watson-Crick (e.g., A-T/U and C-G) or Hoogsteen base pairing between nucleotides or nucleotide analogs of nucleic acid molecules. A full complement or fully complementary may mean 100% complementary base pairing between nucleotides or nucleotide analogs of nucleic acid molecules.

"Stringent hybridization conditions" as used herein refers to conditions under which a first nucleic acid sequence *(e.g.,* probe) hybridizes to a second nucleic acid sequence *(e.g.,* target), such as in a complex mixture of nucleic acids. Stringent conditions are sequence-dependent and different under different circumstances and can be suitably selected by one skilled in the art. Stringent conditions may be selected to be about 5-10°C lower than the thermal melting point (Tm) for the specific sequence at a defined ionic strength pH. The Tm may be the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01-1.0 M sodium ion concentration (or other salts) at pH 7.0 to 8.3 and the temperature is at least about 30°C for short probes *(e.g.,* about 10-50 nucleotides) and at least about 60°C for long probes *(e.g.,* greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5xSSC, and 1% SDS, incubating at 42°C, or, 5xSSC, 1% SDS, incubating at 65°C., with wash in 0.2xSSC, and 0.1% SDS at 65°C. However, several factors other than temperature, such as salt concentration, can influence the stringency of hybridization and one skilled in the art can suitably select the factors to accomplish a similar stringency.

"Hybridization" means the Watson-Crick base-pairing of essentially complementary nucleotide sequences (polymers of nucleic acids) to form a double-stranded molecule.

"Attached" or "immobilized" as used herein to refer to a nucleic acid *(e.g.,* a probe) and a solid support may mean that the binding between the probe and the solid support is sufficient to be stable under conditions of binding, washing, analysis, and removal. The binding may be covalent or non-covalent. Covalent bonds may be formed directly between the probe and the solid support or may be formed by a crosslinker or by the inclusion of a specific reactive group on either the solid support or the probe or both molecules. Non-covalent binding may be one or more of electrostatic, hydrophilic, and hydrophobic interactions. Included in non-covalent binding is the covalent attachment of a molecule, such as streptavidin, to the support and the non-covalent binding of a biotinylated probe to the streptavidin. Immobilization may also involve a combination of covalent and non-covalent interactions.

The terms "determining," "measuring," "assessing," and "assaying" are used interchangeably and include both quantitative and qualitative measurement, and include determining if a characteristic, trait, or feature is present or not. Assessing may be relative or absolute. "Assessing the presence of' a target includes determining the amount of the target present, as well as determining whether it is present or absent.

The term "treating" or "treatment" refers to administration of a compound or agent to a subject who has a disorder or is at risk of developing the disorder with the purpose to cure, alleviate, relieve, remedy, delay the onset of, prevent, or ameliorate the disorder, the symptom of the disorder, the disease state secondary to the disorder, or the predisposition toward the disorder.

As used herein, the term *"in vitro"* refers to events that occur in an artificial environment, *e.g.,* in a test tube or reaction vessel, in cell culture, etc., rather than within a multi-cellular organism.

As used herein, the term *"in vivo"* refers to events that occur within a multi-cellular organism, such as a non-human animal.

It is noted here that, as used in this specification and the appended claims, the singular forms "a," "an," and "the" include plural reference unless the context clearly dictates otherwise.

The terms "including," "comprising," "containing," or "having" and variations thereof are meant to encompass the items listed thereafter and equivalents thereof as well as additional subject matter unless otherwise noted.

The phrases "in one embodiment," "in various embodiments," "in some embodiments," and the like are used repeatedly. Such phrases do not necessarily refer to the same embodiment, but they may unless the context dictates otherwise.

The terms "and/or" or "/" means any one of the items, any combination of the items, or all of the items with which this term is associated.

The word "substantially" does not exclude "completely," *e.g.,* a composition which is "substantially free" from Y may be completely free from Y. Where necessary, the word "substantially" may be omitted from the definition of the invention.

As used herein, the term "approximately" or "about," as applied to one or more values of interest, refers to a value that is similar to a stated reference value. In some embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value). Unless indicated otherwise herein, the term "about" is intended to include values, *e.g*., weight percents, proximate to the recited range that are equivalent in terms of the functionality of the individual ingredient, the composition, or the embodiment.

As disclosed herein, a number of ranges of values are provided. It is understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included in the invention.

As used herein, the term "each," when used in reference to a collection of items, is intended to identify an individual item in the collection but does not necessarily refer to every item in the collection. Exceptions can occur if explicit disclosure or context clearly dictates otherwise.

The use of any and all examples, or exemplary language *(e.g.,* "such as") provided herein, is intended merely to better illuminate the invention and does not pose a limitation on the scope of the invention unless otherwise claimed. No language in the specification should be construed as indicating any non-claimed element as essential to the practice of the invention.

All methods described herein are performed in any suitable order unless otherwise indicated herein or otherwise clearly contradicted by context. In regard to any of the methods provided, the steps of the method may occur simultaneously or sequentially. When the steps of the method occur sequentially, the steps may occur in any order, unless noted otherwise. In cases in which a method comprises a combination of steps, each and every combination or subcombination of the steps is encompassed within the scope of the disclosure, unless otherwise noted herein.

It is understood that the examples and embodiments described herein are for illustrative purposes only and that various modifications or changes in light thereof will be suggested to persons skilled in the art and are to be included within the scope of the appended claims.

### IV. EXAMPLES

### EXAMPLE 1: Impact of APOE genotype on survival in human melanoma in the TCGA-SKCM cohort

To assess APOE genotypes of patients with melanoma, the aligned whole-exome sequencing *BAM* files sliced for the genomic coordinates chr19:44904748-44910394 (GRCh38) from the TCGA-SKCM project using the Genomic Data Commons API were downloaded (Network, T. C. G. A. (2015) Cell, 161(7), 1681-1696). APOE variants were called using the samtools/bcftools package (Li, H. (2011) Bioinformatics, 27(21), 2987-2993), providing allele frequencies for chr19:44908684 (rs429358) and chr19:44908822 (rs7412) as determined in the Atherosclerosis Risk in Communities (ARIC) study as a prior distribution (Blair, C. K., et al. (2005) Neurology, 64(2), 268-276). Variant calls for these two loci were then annotated with APOE alleles in R. Normal tissue samples (either normal blood, normal solid tissue, or normal buccal cells) were available for 470 patients. Patients that exhibited the E2;E4 genotype (n = 5) were excluded from analyses other than genotype frequency assessment. APOE genotype abundance in the normal population is reported as assessed for Caucasian patients in the ARIC study, given that participants were comparable regarding age and ethnicity (Blair, C. K., et al. (2005) Neurology, 64(2), 268-276). To test for differences in genotype distributions χ² tests were employed.

Clinical data including survival times and clinical response were used as recently curated (Liu, J., et al. (2018) Cell, 173(2), 400-416 The R package 'TCGAbiolinks' was used to add clinical data for Breslow depth and Clark level (Colaprico, A., et al. (2016) Nucleic Acids Res, 44(8), e71). To assess the impact of APOE genotypes on survival, Kaplan-Meier survival analysis was performed, and statistical significance tested with the log-rank test for trend using the 'survival' and 'survminer' packages (Therneau, T. M. (2015). A Package for Survival Analysis in S. Retrieved from https://CRAN.R-project.org/package=survival); (Alboukadel, K., et al. (2018) survminer: Drawing Survival Curves using 'ggplot2'. Retrieved from https://CRAN.R-project.org/package=survminer). All analyses were performed using R v3.5 (The R Foundation for Statistical Computing) and RStudio v1.1.3.

### EXAMPLE 2: Animal studies

All animal experiments were conducted in accordance with a protocol approved by the Institutional Animal Care and Use Committee at The Rockefeller University. Human APOE2 (strain #1547), APOE3 (#1548) and APOE4 (#1549) targeted replacement (knock-in) mice were obtained from TACONIC BIOSCIENCES. *Tyrr.CreER; Braf*^{*V600E*/+}*;Pten*^{*lox*/*lox*} mice) (Dankort, D. et al. Nature genetics 41, 544-552 (2009)) were obtained from Jackson laboratories (#013590).

### EXAMPLE 3: Tumor growth studies

To assess the impact of APOE genotype on the growth of syngeneic melanoma 100,000 (YUMM1.7) cells were subcutaneously injected into the flank of 7-10 week old male human APOE targeted replacement mice. For all injections, cells were mixed 1:1 with growth factor reduced Matrigel (356231, CORNING) and injected in a total volume of 100 µL. Tumor size was measured on the indicated days using digital calipers and calculating tumor volume as (small diameter)² × (large diameter) × Π / 6. To assess metastatic progression 100,000 B16F10-TR-shApoE cells were intravenously injected into the tail vein of human ApoE-knock-in mice, and bioluminescence was measured twice a week.

### EXAMPLE 4: Genetically initiated model of melanoma progression

Human APOE targeted replacement (knock-in) mice were crossed with *Tyrr.CreER; Braf*^{*V600E*/+}*;Pten*^{*lox*/*lox*} mice. To induce melanoma, 6-7 weeks old female mice were injected intraperitoneally with 1 mg of Tamoxifen (T5648, Sigma-Aldrich) on three consecutive days. Tamoxifen solution was prepared by dissolving Tamoxifen powder in 100% ethanol at 50°C for five minutes and subsequent tenfold dilution in peanut oil to yield a 10 mg/mL working solution. To assess melanoma burden, dorsal skin samples stretching from ears to hips were harvested on day 35 after induction, depilated with commercial dépilation cream (Nair), washed with water and fixed in 4% PFA. Skins were then scanned, and percentage of pigmented melanoma lesion area was quantified using Cellprofiler v3 (McQuin, C. et al. CellProfiler 3.0: Next-generation image processing for biology. PLoS Blot 16, e2005970 (2018)).

### EXAMPLE 5: Cell lines

Mouse B16F10 melanoma cells were obtained from the American Tissue Type Collection and cultured according to the supplier's conditions. B16F10 cells transduced with a retroviral construct to express luciferase (Therneau, T. M. (2015). A Package for Survival Analysis in S. Retrieved from https://CRAN.R-project.org/package=survival) and shRNA targeting muring Apoe (shRNA clone TRCN0000011799) were described previously (Pencheva, N., et al. (2014) Cell, 156(5), 986-1001). YUMM1.7 cells originally derived from a *Braf*^{*V600E*/+}*;Pten*^{*-*/*-*} mice; *Cdkn2a*mouse melanoma were generously provided by Marcus Bosenberg (Meeth, K., et al. Pigment Cell Melanoma Res 29, 590-597 (2016)). B16F10 cells were cultured in DMEM medium with Pyruvate and Glutamine (11995, GIBCO) supplemented with 10 % FBS (F4135, SIGMA), Penicillin-Streptomycin (15140, GIBCO), and Amphotericin B (17-936E, LONZA). YUMM1.7 cells were cultured in DMEM/F-12 medium with L-Glutamine, and 15mM HEPES (11330, Gibco) supplemented with 10 % FBS, Penicillin-Streptomycin, Amphotericin B, and 1 % non-essential amino acids (111400, GIBCO). Contamination with mycoplasma was ruled out by PCR testing according to standard protocols (Young, L. et al. Nature Protocols 5, 929-934 (2010)).

### EXAMPLE 6: Mouse genotyping

Genotyping of *Tyrr.CreER; Braf*^{*V600E*/+}*;Pten*^{*-*/*-*} mice was performed to distinguish between mouse and human APOE using standard PCR with independent reactions for mouse and human APOE (PCR product lengths of 200bp and approximately 600bp, respectively). In order to distinguish between human APOE alleles, PCR-restriction fragment length polymorphism genotyping (Hixson, J. E. & Vernier, D. T. Journal of lipid research 31, 545-548 (1990)) was used. In brief, a 244bp fragment of APOE was amplified using standard PCR, digested with *Hhal* (R0139S, New England Biolabs), and allele-specific products were resolved on a 15% polyacrylamide gel. The following primers were used for the indicated PCR reactions:

### Tyr:: CreER; Braf^{V600E/+};Pten^{lox/lox} mice

*Cre* transgene forward: 5'- GCG GTC TGG CAG TAA AAA CTA TC - 3' (SEQ ID NO: 4)
*Cre* transgene reverse: 5' - GTG AAA CAG CAT TGC TGT CAC TT - 3' (SEQ ID NO: 5)
*Cre* internal control forward: 5' - CAC GTG GGC TCC AGC ATT- 3' (SEQ ID NO: 6)
*Cre* internal control reverse: 5' - TCA CCA GTC ATT TCT GCC TTT G- 3' (SEQ ID NO: 7)
*Braf* forward: 5' - TGA GTA TTT TTG TGG CAA CTG C - 3' (SEQ ID NO: 8)
*Braf* reverse: 5' - CTC TGC TGG GAA AGC GGC - 3' (SEQ ID NO: 9)
*Pten* forward: 5' - CAA GCA CTC TGC GAA CTG AG - 3' (SEQ ID NO: 10)
*Pten* reverse: 5' - AAG TTT TTG AAG GCA AGA TGC - 3' (SEQ ID NO: 11)
Mouse versus human knock-in APOE mice
Common forward: 5'- TAC CGG CTC AAC TAG GAA CCA T-3' (SEQ ID NO: 12)
Mouse *Apoe* reverse: 5' - TTT AAT CGT CCT CCA TCC CTG C-3' (SEQ ID NO: 13)
Human APOE reverse: 5' - GTT CCA TCT CAG TCC CAG TCTC - 3' (SEQ ID NO: 14)
Human APOE allele restriction length polymorphism
Human APOE forward: 5' - ACA GAA TTC GCC CCG GCC TGG TAC AC - 3' (SEQ ID NO: 15)
Human APOE reverse: 5' - TAA GCT TGG CAC GGC TGT CCA AGG A - 3' (SEQ ID NO: 16)

### EXAMPLE 7: Tumor growth studies and treatments

To assess the impact of APOE genotype on the growth of syngeneic melanoma, 1 × 10⁵ YUMM1.7 cells were subcutaneously injected into the flank of 6-10-weeks-old, sex-matched human APOE targeted replacement mice. Cells were injected in a total volume of 100 µL, and YUMM1.7 cells were mixed 1:1 with growth factor reduced Matrigel (356231, Corning) before injection. Tumor size was measured on the indicated days using digital calipers, and tumor volume was calculated as (small diameter)² × (large diameter) × Π / 6. In experiments employing YUMMER1.7 cells, 5 × 10⁵ cells resuspended in PBS were injected subcutaneously into the flank. For LXR-agonistic treatment, mice were administered chow supplemented with the synthetic LXR-agonist RGX-104 (Rgenix (Tavazoie, M., et al. (2018) Cell, 172(4), 825-840)) at 628.5 mg/kg chow (Research Diets, approximate target dose of 100 mg/kg body weight) starting on day 3 post injection. For anti-PD1 treatment, mice were injected intraperitoneally with 250 µg and 125 µg of anti-PD-1 antibody (BioXCell, clone RMP1-14) on days 6 and 9 post tumor cell injection, respectively. Control mice received PBS injections on the same days. For survival analysis in the YUMMER1.7 model, mice were euthanized when the tumor volume exceeded 1000 mm³. Therapy responses were considered complete (CR, complete response) when tumor volumes fell below 16 mm³ (lowest limit of detection).

### EXAMPLE 8: Analysis of APOE genotype in the TCGA cohorts

The Cancer Genome Atlas (TCGA), were downloaded aligned whole-exome sequencing BAM files sliced for the genomic coordinates chr19:44904748-44910394 (GRCh38) using the Genomic Data Commons API (Network, T. C. G. A. (2015) Cell, 161(7), 1681-1696) to assess APOE genotypes in cancer patients. *APOE* variants using the samtools/bcftools package (Li, H. (2011) Bioinformatics, 27(21), 2987-2993), providing allele frequencies for chr19:44908684 (rs429358) and chr19:44908822 (rs7412) as determined in the Atherosclerosis Risk in Communities (ARIC) study (Blair, C. K., et al. (2005) Neurology, 64(2), 268-276) were called as a prior distribution. Patients that exhibited the APOE2;APOE4 genotype (n = 5) were excluded from analyses except for genotype frequency assessment.

Clinical data including survival times and clinical response were used as recently curated (Liu, J., et al. (2018) Cell, 173(2), 400-416.). The R package `TCGAbiolinks' was used to add clinical data for Breslow depth and Clark level (Colaprico, A., et al. (2016) Nucleic Acids Res, 44(8), e71). To assess the impact of APOE genotypes on survival, Kaplan-Meier survival analyses were performed, and statistical significance was assessed with the log-rank test using the 'survival' and 'survminer' packages (Therneau, T. M. (2015) A Package for Survival Analysis in S. Retrieved from https://CRAN.R-project.org/package=survival); (Alboukadel, K., et al. (2018) survminer: Drawing Survival Curves using 'ggplot2'. Retrieved from https://CRAN.R-project.org/package=survminer). Hazard ratios were calculated according to a Cox proportional hazards regression model using the 'survival' R package (Therneau, T. M. (2015) A Package for Survival Analysis in S. Retrieved from https://CRAN.R-project.org/package=survival). For visualization purposes, survival data were truncated at 12 years. All analyses were performed using R v3.5 (The R Foundation for Statistical Computing) and RStudio v1.1.3.

### EXAMPLE 9: Analysis of APOE genotype in the anti-PD1 melanoma studies

Analyses of the Roh *et al.* (Roh, W., et al. (2017) Sci Transl Med, 9(379).) and Riaz *et al.* (Riaz, N., et al. (2017) Cell, 171(4), 934-949) cohorts were performed as described for the TCGA-SKCM cohort. In brief, normal tissue whole-exome sequencing data were downloaded from dbGaP (BioProject IDs PRJNA369259 and PRJNA359359), and APOE genotype was called as detailed above. No genotype could be determined for one patient in the Roh *et al.* cohort. For the Roh *et al.* cohort, only patients that received both anti-CTLA4 and anti-PD1 treatment were considered. In the Riaz *et al.* cohort patients were stratified by prior CTLA4 treatment status. Kaplan-Meier survival analyses were performed using the 'survival' and 'survminer' packages, as detailed above.

### EXAMPLE 10: APOE genotype modulates cancer survival in the context of immunotherapies

To assess whether APOE genotype could impact melanoma progression in the context of immunotherapy, the effect of APOE genotype on progression of the immunogenic YUMMER1.7 melanoma model was analyzed, which is susceptible to anti-PD1 checkpoint therapy (Wang, J., et al. (2017) Pigment Cell & Melanoma Research, 30(4), 428-435). APOE4 mice survived significantly longer than APOE2 mice upon anti-PD1 treatment, suggesting that APOE genotype modulates melanoma outcome also in the context of immunotherapy (FIGs. 4a-b). To assess whether this effect could also be observed in humans, melanoma patients that received anti-PD1 checkpoint inhibition therapy after progressing on anti-CTLA4 checkpoint blockade were analyzed (Roh, W., et al. (2017) Sci Transl Med, 9(379)). Indeed, APOE genotype was significantly associated with survival in this cohort (p = 0.017, log-rank test). Consistent with observations on survival of earlier stage patients described above, APOE4 and APOE2 carriers exhibited the longest and shortest survival upon anti-PD1 therapy, respectively (FIG. 4c). These findings were validated in an independent cohort of patients receiving anti-PD1 therapy upon progressing on CTLA4 blockade (Riaz, N., et al. (2017) Cell, 171(4), 934-949). Consistent with the cohort studied by Roh, W., *et al.* APOE4 and APOE2 carriers in this study by Riaz *et al.* also exhibited the longest and shortest survival outcomes, respectively (FIG. 4d). Thus, in both of these cohorts, APOE genotype associated with survival in patients who had progressed on prior immunotherapy.

Pharmacologic activation of APOE may also augment the differential impact of APOE variants on melanoma progression. Liver-X-receptors (LXR) are nuclear hormone receptors that transcriptionally activate a number of genes implicated in cholesterol and lipid metabolism, including APOE (Evans, R.M., et al. (2014) Cell, 157(1), 255-266; Hong, C., et al. (2014) Nat Rev Drug Discov, 13(6), 433-444). LXR agonism has been shown to enhance anti-tumor immunity-an effect shown to be primarily mediated by APOE (Tavazoie, M., et al. (2018) Cell, 172(4), 825-840.e18; Pencheva, N., et al. (2014) Cell, 156(5), 986-1001). Indeed, the efficacy of LXR agonism was completely abrogated in APOE2 mice, while APOE4 mice benefitted from robust anti-tumor effects of treatment, augmenting the differences in tumor progression between APOE2 and APOE4 mice (FIG. 4e-fs). Thus, distinct APOE genotypes elicited differential responsiveness to LXR agonistic therapy and may serve as potential biomarkers for current clinical efforts investigating the use of LXR agonism in cancer therapy (Tavazoie, M., et al. (2018) Cell, 172(4), 825-840).

### EXAMPLE 11: APOE genotype modulates outcome of thyroid and breast cancer

To assess the impact of APOE genotype on outcome of patients with thyroid and breast cancer, normal-tissue whole-exome sequencing data from patients with stage II/III disease from The Cancer Genome Atlas (TCGA) cohorts were analyzed. In both cohorts, APOE genotype was significantly associated with survival (FIGs. 5a-b), with patients carrying the APOE2 genotype exhibiting the shortest survival.

### EXAMPLE 12: The impact of APOE genotype on breast cancer survival interacts with tumor immune-cell infiltration

Analysis of the entire TCGA breast cancer cohort revealed patients carrying either the APOE2 or the APOE4 allele to exhibit shorter survival than APOE3 homozygotes (FIGs. 6a-c). In light of this difference to the melanoma and thyroid cancer cohorts, the impact of APOE on breast cancer survival may be modulated by other factors determining cancer outcome. Given the impact of APOE on tumor immunity, an interaction of the degree of immune cell infiltration (as assessed by tumor transcriptome deconvolution (Thorsson, V., et al. (2018) Immunity, 48(4), 812-830.e14.) and APOE genotype was tested. Immune infiltration interacted with APOE genotype, whereby in both groups of patients with low and high leukocyte infiltration there was a significant association of APOE genotype and overall survival, but the survival of APOE2 and APOE4 carriers was reversed between the two groups. It was therefore concluded that the impact of APOE genotype on breast cancer survival interacts with immune cell infiltration of the tumor.

### EXAMPLE 13: Statistical analysis

Unless otherwise noted all data are expressed as mean ± standard error of the mean and groups were compared using t-tests for normally distributed data or Mann Whitney tests for non-normally distributed data as indicated in the figure legends. A significant difference was concluded at *p* < 0.05. To compare survival times the log-rank test was employed. Throughout all figures: **p* < 0.05, ***p* < 0.01, ****p* < 0.001, and **"*"p* < 0.0001.

Other objects, features, and advantages of the present invention will become apparent from the following detailed description. It should be understood, however, that the detailed description and the examples, while indicating specific embodiments of the invention, are given by way of illustration only. Additionally, it is contemplated that changes and modifications within the scope of the invention will become apparent to those skilled in the art from this detailed description.

### EXAMPLE 14

RGX-104 was administered to patients with solid cancers as a monotherapy or in combination with either docetaxel, nivolumab, or ipilimumab. Fifty-one patients were evaluable for both radiographic assessment of tumor response and ApoE genotype. Below is a table showing the number of patients with each genotype and the number of clinical responses (Partial Responses) to the therapy.

| ApoE genotype | Number of patients | Number of Partial Responses | Percent of patients with a Partial Response |
|---|---|---|---|
| E2/E4 | 3 | 1 | 33.3% |
| E3/E4 | 8 | 1 | 12.5% |
| E3/E3 | 30 | 1 | 3.3% |
| E2/E3 | 10 | 0 | 0 % |

## Claims

1. An LXRβ agonist, or a pharmaceutically acceptable salt thereof, for use in a method of treating cancer in a subject having at least one ε4 allele from polymorphisms at rs7412 or rs429358 or ε3 alleles from polymorphisms at rs7412 and rs429358 in the *ApoE* gene on chromosome 19, the method comprising administering an effective amount of the LXRβ agonist, or a pharmaceutically acceptable salt thereof, to the subject, preferably wherein the LXRβ agonist is 2-[3-[(3R)-3-[[2-chloro-3-(trifluoromethyl)phenyl]methyl-(2,2-diphenylethyl)amino]butoxy]phenyl]acetic acid, or a pharmaceutically acceptable salt thereof.

2. An LXRβ agonist, or a pharmaceutically acceptable salt thereof, for use in a method of treating cancer in a subject in need thereof, the method comprising:
(a) providing a subject identified as having at least one ε4 allele from polymorphisms at rs7412 or rs429358 or ε3 alleles from polymorphisms at rs7412 and rs429358 in the *ApoE* gene on chromosome 19, preferably wherein the subject is identified as likely to benefit from treatment with the LXRβ agonist, or a pharmaceutically acceptable salt thereof, based on the presence of the at least one ε4 allele from polymorphisms at rs7412 or rs429358 or ε3 alleles from polymorphisms at rs7412 and rs429358; and
(b) administering to the subject an effective amount of the LXRβ agonist, or a pharmaceutically acceptable salt thereof, preferably wherein the LXRβ agonist is 2-[3-[(3R)-3-[[2-chloro-3-(trifluoromethyl)phenyl]methyl-(2,2-diphenylethyl)amino]butoxy]phenyl]acetic acid, or a pharmaceutically acceptable salt thereof.

3. An LXRβ agonist, or a pharmaceutically acceptable salt thereof, for use in a method for treating cancer in a subject, the method comprising:
(a) obtaining a sample containing nucleic acid from the subject;
(b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19;
(c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with the LXRβ agonist, or a pharmaceutically acceptable salt thereof; and
(d) administering an effective amount of the LXRβ agonist, or a pharmaceutically acceptable salt thereof, to the identified subject, preferably wherein the LXRβ agonist is 2-[3-[(3R)-3-[[2-chloro-3-(trifluoromethyl)phenyl]methyl-(2,2-diphenylethyl)amino]butoxy]phenyl]acetic acid, or a pharmaceutically acceptable salt thereof,
whereby the cancer is treated.

4. A method of identifying an individual having a cancer who may benefit from treatment with an LXRβ agonist, or a pharmaceutically acceptable salt thereof, the method comprising:
(a) obtaining a sample containing nucleic acid from the subject;
(b) performing a genotyping assay on the sample and detecting the presence of at least one ε4 allele from polymorphisms at rs7412 or rs429358 or ε3 alleles from polymorphisms at rs7412 and rs429358 in the *ApoE* gene on chromosome 19; and
(c) identifying the subject having the at least one ε4 allele from polymorphisms at rs7412 or rs429358 or ε3 alleles from polymorphisms at rs7412 and rs429358 in the *ApoE* gene on chromosome 19 as having an increased likelihood of benefiting from treatment with the LXRβ agonist, or a pharmaceutically acceptable salt thereof,
thereby identifying the individual having a cancer who may benefit from treatment with an LXRβ agonist, or a pharmaceutically acceptable salt thereof,
preferably wherein the LXRβ agonist is 2-[3-[(3*R*)-3-[[2-chloro-3-(trifluoromethyl)phenyl]methyl-(2,2-diphenylethyl)amino]butoxy]phenyl]acetic acid, or a pharmaceutically acceptable salt thereof.

5. The LXRβ agonist, or a pharmaceutically acceptable salt thereof, for the use of any one of claims 1 to 3, , wherein the presence of at least one ε4 allele from polymorphisms at rs7412 or rs429358 or ε3 alleles from polymorphisms at rs7412 and rs429358 in the *ApoE* gene on chromosome 19 is determined by Polymerase Chain Reaction (PCR), or wherein the presence of at least one ε4 allele from polymorphisms at rs7412 or rs429358 or ε3 alleles from polymorphisms at rs7412 and rs429358 in the *ApoE* gene on chromosome 19 is determined by the expression level of APOE3 protein or APOE4 protein in the subject,
preferably wherein:
(a) the cancer is breast cancer, colon cancer, renal cell cancer, lung cancer, hepatocellular carcinoma, gastric cancer, ovarian cancer, pancreatic cancer, esophageal cancer, prostate cancer, sarcoma, bladder cancer, head and neck cancer, glioblastoma, diffuse large B-cell lymphoma, leukemia, or melanoma, further preferably wherein the cancer is melanoma, small cell lung cancer, or non-small cell lung cancer,
(b) the cancer is metastatic and/or locally advanced,
(c) the cancer is unresectable,
(d) the effective amount comprises an amount effective to suppress metastatic colonization of the cancer,
(e) the method further comprises administering an additional anti-cancer therapy to the subject, preferably wherein the additional anti-cancer therapy comprises surgery, radiation, chemotherapy, and/or immunotherapy, further preferably wherein the additional anti-cancer therapy comprises chemotherapy and/or immunotherapy, and further preferably wherein the chemotherapy comprises docetaxel, ipilimumab, carboplatin or cisplatin, pemetrexed, and/or pembrolizumab,
(f) the method further comprises administering to the subject an effective amount of folic acid, vitamin B12, corticosteroids, a statin, an anti-emetic agent, an anti-diarrheal agent, an appetite stimulant, a general stimulant, a bisphosphonate, a gonadotrophin-releasing hormone agonist, growth factors, and/or an LHRH agonist,
(g) the subject has ε3 alleles from polymorphisms at rs7412 and rs429358 in the *ApoE* gene,
(h) the subject has at least one ε4 allele from polymorphisms at rs7412 or rs429358 in the *ApoE* gene, or
(i) the subject has ε4 alleles from polymorphisms at rs7412 and rs429358, an ε3 allele and an ε4 allele from polymorphisms at rs7412 and rs429358, or an ε2 and an ε4 allele from polymorphisms at rs7412 and rs429358 in the *ApoE* gene, further preferably wherein the subject has ε4 alleles from polymorphisms at rs7412 and rs429358 in the *ApoE* gene.

## Patentansprüche

1. LXRβ-Agonist oder ein pharmazeutisch akzeptables Salz davon zur Verwendung in einem Verfahren zur Behandlung von Krebs bei einem Patienten mit mindestens einem ε4-Allel aus Polymorphismen bei rs7412 oder rs429358 oder ε3-Allelen aus Polymorphismen bei rs7412 und rs429358 im *ApoE-Gen* auf Chromosom 19, wobei das Verfahren die Verabreichung einer wirksamen Menge des LXRβ-Agonisten oder eines pharmazeutisch annehmbaren Salzes davon an das Subjekt umfasst, wobei der LXRβ-Agonist vorzugsweise 2-[3-[(3R)-3-[[2-Chlor-3-(trifluormethyl)phenyl]methyl-(2,2-diphenylethyl)amino]butoxy]phenyl]essigsäure oder ein pharmazeutisch annehmbares Salz davon ist.

2. LXRβ-Agonist oder ein pharmazeutisch akzeptables Salz davon zur Verwendung in einem Verfahren zur Behandlung von Krebs bei einem Patienten, der dessen bedarf, wobei das Verfahren umfasst:
(a) Bereitstellen eines Patienten, der als Träger von mindestens einem ε4-Allel aus Polymorphismen bei rs7412 oder rs429358 oder ε3-Allelen aus Polymorphismen bei rs7412 und rs429358 im *ApoE-Gen* auf Chromosom 19 identifiziert wurde, wobei das Subjekt vorzugsweise auf der Grundlage des Vorhandenseins mindestens eines ε4-Allels aus den Polymorphismen bei rs7412 oder rs429358 oder ε3-Allelen aus den Polymorphismen bei rs7412 und rs429358 als wahrscheinlich von einer Behandlung mit dem LXRβ-Agonisten oder einem pharmazeutisch akzeptablen Salz davon profitierend identifiziert wird; und
(b) Verabreichung einer wirksamen Menge des LXRβ-Agonisten oder eines pharmazeutisch akzeptablen Salzes davon an das Patienten, wobei es sich bei dem LXRβ-Agonisten vorzugsweise um 2-[3-[(3R)-3-[[2-Chlor-3-(trifluormethyl)phenyl]methyl-(2,2-diphenylethyl)amino]butoxy]phenyl]essigsäure oder ein pharmazeutisch akzeptables Salz davon handelt.

3. LXRβ-Agonist oder ein pharmazeutisch annehmbares Salz davon zur Verwendung in einem Verfahren zur Behandlung von Krebs bei einem Patienten, wobei das Verfahren umfasst:
(a) Erhalten einer Nukleinsäure enthaltenden Probe von dem Patienten;
(b) Durchführen eines Genotypisierungstests an der Probe und Nachweisen des Vorhandenseins von mindestens einem ε4-Allel aus Polymorphismen bei rs7412 oder rs429358 oder ε3-Allelen aus Polymorphismen bei rs7412 und rs429358 im ApoE-Gen auf Chromosom 19;
(c) Identifizierung des Patienten mit mindestens einem ε4-Allel aus den Polymorphismen bei rs7412 oder rs429358 oder ε3-Allelen aus den Polymorphismen bei rs7412 und rs429358 im *ApoE-Gen* auf Chromosom 19 als Person, die eine erhöhte Wahrscheinlichkeit hat, von der Behandlung mit dem LXRβ-Agonisten oder einem pharmazeutisch akzeptablen Salz davon zu profitieren; und
(d) Verabreichung einer wirksamen Menge des LXRβ-Agonisten oder eines pharmazeutisch akzeptablen Salzes davon an das identifizierte Subjekt, wobei es sich bei dem LXRβ-Agonisten vorzugsweise um 2-[3-[(3R)-3-[[2-Chlor-3-(trifluormethyl)-phenyl]-methyl-(2,2-diphenylethyl)-amino]-butoxy]-phenyl]-essigsäure oder ein pharmazeutisch akzeptables Salz davon handelt, wodurch der Krebs behandelt wird.

4. Verfahren zur Identifizierung eines Individuums mit Krebs, das von einer Behandlung mit einem LXRβ-Agonisten oder einem pharmazeutisch akzeptablen Salz davon profitieren kann, wobei das Verfahren umfasst:
(a) Erhalten einer Nukleinsäure enthaltenden Probe von dem Patienten;
(b) Durchführung eines Genotypisierungstests an der Probe und Nachweis des Vorhandenseins von mindestens einem ε4-Allel aus Polymorphismen bei rs7412 oder rs429358 oder ε3-Allelen aus Polymorphismen bei rs7412 und rs429358 im *ApoE-Gen* auf Chromosom 19; und
(c) Identifizierung des Subjekts, das mindestens ein ε4-Allel aus den Polymorphismen bei rs7412 oder rs429358 oder ε3-Allele aus den Polymorphismen bei rs7412 und rs429358 im *ApoE-Gen* auf Chromosom 19 aufweist, als Person, die eine erhöhte Wahrscheinlichkeit hat, von der Behandlung mit dem LXRβ-Agonisten oder einem pharmazeutisch akzeptablen Salz davon zu profitieren,
wodurch die Person mit einem Krebs identifiziert wird, die von einer Behandlung mit einem LXRβ-Agonisten oder einem pharmazeutisch akzeptablen Salz davon profitieren kann,
wobei der LXRβ-Agonist vorzugsweise 2-[3-[(3R)-3-[[2-Chlor-3-(trifluormethyl)-phenyl]-methyl-(2,2-diphenylethyl)-amino]-butoxy]-phenyl]-essigsäure oder ein pharmazeutisch akzeptables Salz davon ist.

5. LXRβ-Agonist oder ein pharmazeutisch annehmbares Salz davon zur Verwendung nach einem der Ansprüche 1 bis 3, wobei das Vorhandensein von mindestens einem ε4-Allel aus Polymorphismen bei rs7412 oder rs429358 oder ε3-Allelen aus Polymorphismen bei rs7412 und rs429358 im *ApoE-Gen* auf Chromosom 19 durch Polymerase-Kettenreaktion (PCR) bestimmt wird, oder wobei das Vorhandensein mindestens eines ε4-Allels aus den Polymorphismen bei rs7412 oder rs429358 oder ε3-Allelen aus den Polymorphismen bei rs7412 und rs429358 im *ApoE-Gen* auf Chromosom 19 durch die Expressionsmenge des APOE3-Proteins oder des APOE4-Proteins in der Person bestimmt wird,
wobei vorzugsweise:
(a) der Krebs Brustkrebs, Dickdarmkrebs, Nierenzellkrebs, Lungenkrebs, hepatozelluläres Karzinom, Magenkrebs, Eierstockkrebs, Bauchspeicheldrüsenkrebs, Speiseröhrenkrebs, Prostatakrebs, Sarkom, Blasenkrebs, Kopf- und Halskrebs, Glioblastom, diffuses großzelliges B-Zell-Lymphom, Leukämie oder Melanom ist, wobei der Krebs vorzugsweise Melanom, kleinzelliger Lungenkrebs oder nichtkleinzelliger Lungenkrebs ist,
(b) der Krebs ist metastasiert und/oder lokal fortgeschritten,
(c) der Krebs ist nicht resektabel,
(d) die wirksame Menge umfasst eine Menge, die wirksam ist, um die metastatische Besiedlung des Krebses zu unterdrücken,
(e) das Verfahren ferner die Verabreichung einer zusätzlichen Anti-Krebs-Therapie an das Subjekt umfasst, wobei die zusätzliche Anti-Krebs-Therapie vorzugsweise eine Operation, Bestrahlung, Chemotherapie und/oder Immuntherapie umfasst, wobei die zusätzliche Anti-Krebs-Therapie vorzugsweise eine Chemotherapie und/oder Immuntherapie umfasst, und wobei die Chemotherapie vorzugsweise Docetaxel, Ipilimumab, Carboplatin oder Cisplatin, Pemetrexed und/oder Pembrolizumab umfasst,
(f) das Verfahren umfasst ferner die Verabreichung einer wirksamen Menge an Folsäure, Vitamin B12, Kortikosteroiden, einem Statin, einem Antiemetikum, einem Mittel gegen Durchfall, einem Appetitstimulans, einem allgemeinen Stimulans, einem Bisphosphonat, einem Gonadotropin-Releasing-Hormon-Agonisten, Wachstumsfaktoren und/oder einem LHRH-Agonisten an den Patienten,
(g) der Proband hat ε3-Allele von Polymorphismen bei rs7412 und rs429358 im *ApoE-Gen,*
(h) der Proband mindestens ein ε4-Allel von Polymorphismen an rs7412 oder rs429358 im *ApoE-Gen* aufweist oder
(i) der Proband hat ε4 Allele aus Polymorphismen bei rs7412 und rs429358, ein ε3-Allel und ein ε4-Allel aus Polymorphismen bei rs7412 und rs429358, oder ein ε2-Allel und ein ε4-Allel aus Polymorphismen an rs7412 und rs429358 im *ApoE-Gen,* weiter bevorzugt, wobei das Subjekt ε4-Allele aus Polymorphismen an rs7412 und rs429358 im *ApoE-Gen* hat.

## Revendications

1. Agoniste LXRβ, ou un sel pharmaceutiquement acceptable de celui-ci, destiné à être utilisé dans un procédé de traitement du cancer chez un sujet présentant au moins un allèle ε4 issu de polymorphismes au niveau de rs7412 ou rs429358 ou des allèles ε3 issus de polymorphismes au niveau de rs7412 et rs429358 dans le gène *ApoE* sur le chromosome 19, le procédé comprenant l'administration d'une quantité efficace de l'agoniste LXRβ, ou d'un sel pharmaceutiquement acceptable de celui-ci, au sujet, préférablement dans lequel l'agoniste LXRβ est l'acide 2-[3-[(3R)-3-[[2-chloro-3-(trifluorométhyl)phényl]méthyl-(2,2-diphényléthyl)amino]butoxy]phényl]acétique, ou un sel pharmaceutiquement acceptable de celui-ci.

2. Agoniste LXRβ, ou un sel pharmaceutiquement acceptable de celui-ci, destiné à être utilisé dans un procédé de traitement du cancer chez un sujet en ayant besoin, le procédé comprenant :
(a) la fourniture à un sujet identifié comme ayant au moins un allèle ε4 issu de polymorphismes au niveau de rs7412 ou rs429358 ou des allèles ε3 issus de polymorphismes au niveau de rs7412 et rs429358 dans le gène *ApoE* sur le chromosome 19, préférablement dans lequel le sujet est identifié comme susceptible de bénéficier d'un traitement avec l'agoniste LXRβ, ou un sel pharmaceutiquement acceptable de celui-ci, sur la base de la présence d'au moins un allèle ε4 issu de polymorphismes au niveau de rs7412 ou rs429358 ou des allèles ε3 issus de polymorphismes au niveau de rs7412 et rs429358 ; et
(b) l'administration au sujet d'une quantité efficace de l'agoniste LXRβ, ou d'un sel pharmaceutiquement acceptable de celui-ci, préférablement dans lequel l'agoniste LXRβ est l'acide 2-[3-[(3R)-3-[[2-chloro-3-(trifluorométhyl)phényl]méthyl-(2,2-diphényléthyl)amino]butoxy]phényl] acétique, ou un sel pharmaceutiquement acceptable de celui-ci.

3. Agoniste LXRβ, ou un sel pharmaceutiquement acceptable de celui-ci, destiné à être utilisé dans un procédé de traitement du cancer chez un sujet, le procédé comprenant :
(a) l'obtention d'un échantillon contenant de l'acide nucléique du sujet ;
(b) la réalisation d'un test de génotypage sur l'échantillon et la détection de la présence d'au moins un allèle ε4 issu de polymorphismes au niveau de rs7412 ou rs429358 ou d'allèles ε3 issus de polymorphismes au niveau de rs7412 et rs429358 dans le gène *ApoE* sur le chromosome 19 ;
(c) l'identification du sujet possédant au moins un allèle ε4 issu de polymorphismes au niveau de rs7412 ou rs429358 ou des allèles ε3 issus de polymorphismes au niveau de rs7412 et rs429358 dans le gène *ApoE* sur le chromosome 19 comme ayant une probabilité accrue de bénéficier d'un traitement avec l'agoniste LXRβ, ou un sel pharmaceutiquement acceptable de celui-ci ; et
(d) l'administration d'une quantité efficace de l'agoniste LXRβ, ou d'un sel pharmaceutiquement acceptable de celui-ci, au sujet identifié, préférablement dans lequel l'agoniste LXRβ est l'acide 2-[3-[(3R)-3-[[2-chloro-3-(trifluorométhyl)phényl]méthyl-(2,2-diphényléthyl)amino]butoxy]phényl]acétique, ou un sel pharmaceutiquement acceptable de celui-ci, par lequel le cancer est traité.

4. Procédé d'identification d'un individu atteint d'un cancer qui pourrait bénéficier d'un traitement avec un agoniste LXRβ, ou un sel pharmaceutiquement acceptable de celui-ci, le procédé comprenant :
(a) l'obtention d'un échantillon contenant de l'acide nucléique du sujet ;
(b) la réalisation d'un test de génotypage sur l'échantillon et la détection de la présence d'au moins un allèle ε4 issu de polymorphismes au niveau de rs7412 ou rs429358 ou d'allèles ε3 issus de polymorphismes au niveau de rs7412 et rs429358 dans le gène *ApoE* sur le chromosome 19 ; et
(c) l'identification du sujet possédant au moins un allèle ε4 issu de polymorphismes au niveau de rs7412 ou rs429358 ou des allèles ε3 issus de polymorphismes au niveau de rs7412 et rs429358 dans le gène *ApoE* sur le chromosome 19 comme ayant une probabilité accrue de bénéficier d'un traitement avec l'agoniste LXRβ, ou un sel pharmaceutiquement acceptable de celui-ci,
identifiant ainsi l'individu atteint d'un cancer qui pourrait bénéficier d'un traitement avec un agoniste LXRβ, ou un sel pharmaceutiquement acceptable de celui-ci,
préférablement dans lequel l'agoniste LXRβ est l'acide 2-[3-[(3R)-3-[[2-chloro-3-(trifluorométhyl)phényl]méthyl-(2,2-diphényléthyl)amino]butoxy]phényl]acétique, ou un sel pharmaceutiquement acceptable de celui-ci.

5. Agoniste LXRβ, ou un sel pharmaceutiquement acceptable de celui-ci, pour l'utilisation selon l'une quelconque des revendications 1 à 3, dans lequel la présence d'au moins un allèle ε4 issu de polymorphismes au niveau de rs7412 ou rs429358 ou d'allèles ε3 issu de polymorphismes au niveau de rs7412 et rs429358 dans le gène *ApoE* sur le chromosome 19 est déterminée par réaction en chaîne par polymérase (PCR), ou dans lequel la présence d'au moins un allèle ε4 issu de polymorphismes au niveau de rs7412 ou rs429358 ou d'allèles ε3 issu de polymorphismes au niveau de rs7412 et rs429358 dans le gène *ApoE* sur le chromosome 19 est déterminée par le niveau d'expression de la protéine APOE3 ou de la protéine APOE4 chez le sujet,
préférablement dans lequel :
(a) le cancer est un cancer du sein, un cancer du côlon, un cancer des cellules rénales, un cancer du poumon, un carcinome hépatocellulaire, un cancer gastrique, un cancer de l'ovaire, un cancer du pancréas, un cancer de l'œsophage, un cancer de la prostate, un sarcome, un cancer de la vessie, un cancer de la tête et du cou, un glioblastome, un lymphome diffus à grandes cellules B, une leucémie ou un mélanome, préférablement encore dans lequel le cancer est un mélanome, un cancer du poumon à petites cellules ou un cancer du poumon non à petites cellules,
(b) le cancer est métastatique et/ou localement avancé,
(c) le cancer est non résécable,
(d) la quantité efficace comprend une quantité efficace pour supprimer la colonisation métastatique du cancer,
(e) le procédé comprend en outre l'administration d'une thérapie anticancéreuse supplémentaire au sujet, préférablement dans lequel la thérapie anticancéreuse supplémentaire comprend une intervention chirurgicale, une radiothérapie, une chimiothérapie et/ou une immunothérapie, préférablement encore dans lequel la thérapie anticancéreuse supplémentaire comprend une chimiothérapie et/ou une immunothérapie, et préférablement encore dans lequel la chimiothérapie comprend du docétaxel, de l'ipilimumab, du carboplatine ou du cisplatine, du pémétrexed et/ou du pembrolizumab,
(f) le procédé comprend en outre l'administration au sujet d'une quantité efficace d'acide folique, de vitamine B12, de corticostéroïdes, d'une statine, d'un agent antiémétique, d'un agent antidiarrhéique, d'un stimulant de l'appétit, d'un stimulant général, d'un bisphosphonate, d'un agoniste de l'hormone de libération des gonadotrophines, de facteurs de croissance et/ou d'un agoniste de la LHRH,
(g) le sujet possède des allèles ε3 issus des polymorphismes au niveau de rs7412 et rs429358 dans le gène *ApoE,*
(h) le sujet possède au moins un allèle ε4 issu des polymorphismes au niveau de rs7412 ou rs429358 dans le gène *ApoE,* ou
(i) le sujet possède des allèles ε4 issus de polymorphismes au niveau de rs7412 et rs429358, un allèle ε3 et un allèle ε4 issus de polymorphismes au niveau de rs7412 et rs429358, ou un allèle ε2 et un allèle ε4 issus de polymorphismes au niveau de rs7412 et rs429358 dans le gène *ApoE,* préférablement encore, le sujet possédant des allèles ε4 issus de polymorphismes au niveau de rs7412 et rs429358 dans le gène *ApoE.*
